# EUROPEAN PATENT APPLICATION

(11) **EP 1 905 824 A1**
(43) Date of publication of application: **02.04.2008**
(21) Application number: 06756855.0
(22) Date of filing: 31.05.2006
(51) Int. Cl.: C12N 5/10, C12N 15/09, C12Q 1/02, C12Q 1/66, C12N 9/99, G01N 33/15, G01N 33/50

(54) **A TRANSGENIC CELL AND A METHOD FOR CELL ANALYSIS**

(30) Priority: 31.05.2005 JP 2005160621; 31.05.2005 JP 2005160685
(71) Applicant: Olympus Corporation, Tokyo 151-0072 (JP)
(72) Inventor: YAMAGUCHI, Yuri, Saitama-shi, Saitama 330-0072 (JP); HAYASHI, Shinichi, Sendai-shi, Miyagi 980-0861 (JP); SHIMADA, Yoshihiro, Sagamihara-shi, Kanagawa 229-1123 (JP); TAKAGI, Kosuke, Kawagoe-shi, Saitama 350-0817 (JP); SAKAMOTO, Hiroko, Tokyo 141-0022 (JP)
(74) Representative: Wibbelmann, Jobst
(86) International application number: PCT/JP2006/310935
(87) International publication number: WO 2006/129735

(57) **Abstract**

The transgenic cell of the present invention is **characterized in that** a reporter vector containing a gene coding a reporter protein and an estrogen responsive element (ERE) at the upstream of the promoter region of the reporter protein, wherein the expression of the reporter protein is substantially controlled by the estrogen responsive element is stably introduced.

SEQUENCE LISTING
<110> OLYMPUS CORPORATION
<120> A transgenic cell and a method for cell analysis
<130>
<160> 4
<210> 1
<211> 16
<212> DNA
<213> Artificial Sequence
<220>
<221> misc_feature
<222> (7)..(9)
<223> n stands for any base
<220>
<223> Estrogen responsive element consensus sequence
<400> 1
aggtcannnt gacctt 16

<210> 2
<211> 29
<212> DNA
<213> Artificial Sequence
<220>
<223> Estrogen responsive element
<400> 2
agctaggtca ggatgaccta gctacagct 29

<210> 3
<211> 158
<212> DNA
<213> Artificial Sequence

<220>
<223> HSV-TK promoter sequence
<400> 3
ggccccgccc agcgtcttgt cattggcgaa ttcgaacacg cagatgcagt cggggcggcg
60
cggtcccagg tccacttcgc atattaaggt gacgcgtgtg gcctcgaaca ccgagcgacc
120
ctgcagcgac ccgcttaaca gcgtcaacag cgtgccgc                    158

<210> 4
<211> 314
<212> DNA
<213> Artificial Sequence
<220>
<223> DNA fragment containing estrogen responsive element and
HSV-TK promoter sequence
<400> 4
ctggcgaaag ggggatgtgc tgcaaggcga ttaagttggg taacgccagg gttttcccag
60
tcacgacgtt gtaaaacgac ggccagtgcc aagctaggtc aggatgacct agctacagct
120
tgcatgcctg caggtcgact ctagaggatc cggccccgcc cagcgtcttg tcattggcga
180
attcgaacac gcagatgcag tcggggcggc gcggtcccag gtccacttcg catattaagg
240
tgacgcgtgt ggcctcgaac accgagcgac cctgcagcga cccgcttaac agcgtcaaca
300
gcgtgccgca gatc                                    314

## Description

### FIELD OF THE INVENTION

The present invention relates to a transgenic cell, a method for assessing estrogen signals using the transgenic cell, a method for analysis of inhibitors using the method for assessing, and a method for cell analysis, and particularly to a transgenic cell using a reporter gene, a method for assessing estrogen signals using the transgenic cell, a method for analysis of inhibitors using the method for assessing, and a method for cell analysis using a reporter gene.

### BACKGROUND OF THE INVENTION

Hormones are substances that are produced and secreted by particular cells (mainly endocrine cells and some neurons) in response to in vivo and ex vivo signals, which transfer the signals to other cells via biological fluids. There are three major hormone groups: peptide hormones such as insulin and glucagons, steroid hormones such as androgen and estrogen, and amine hormones such as adrenaline. All hormones bind to a hormone receptor of a target cell to change its physical property, thereby inducing some changes within the cell. Amine and peptide hormones mainly act on the cell membrane to control the intracellular metabolism through functional changes of the cell membrane or modification in production of secondary messengers. Steroid hormones mainly bind to a receptor within the cytoplasm or nuclei to control the expression of genetic information.

Hormonal information is an important factor for ensuring normal life of organisms, including functional expressions and sustained homeostasis.

Hormonal information involves various reactions and enzymes and, in some cases, is intricately controlled via cascade reaction to sustain in vivo homeostasis.

Among above-mentioned hormones, estrogen is a female hormone deeply involved in the development and growth of breast cancer. Breast cancer has the highest prevalence rate in females worldwide and a cure for breast cancer is urgently needed. Many breast cancers are hormone dependent tumors, and growth of breast cancers is hormone (estrogen)-dependent. When estrogen is received to breast cancer cells from an external source, signaling cascades relating to cell proliferation are activated via estrogen receptors (ER). Consequently, the cell proliferation is promoted and development of the cancer occurs. Therefore, in assessment of the pathologic condition of breast cancer, it is significantly useful to analyze the activation of signaling via estrogen receptors (referred to as estrogen signal hereafter) in breast cancer tissue.
Currently, the first option in drug therapy for breast cancer is so-called hormone therapy in which an anti-estrogenic drug, typically tamoxifen or an estrogen antagonist, is administered. The estrogen antagonist binds to estrogen receptors in the cancer cells. However, unlike native estrogen, the estrogen antagonist does not activate the signaling cascade relating to the cell proliferation, which leads to an anti-tumor effect.
Currently, the applicability of hormone therapy to breast cancer cases is assessed by determining whether or not estrogen receptors (ER) are expressed in the cancer cells of the breast cancer tissue of each case, and the therapy is applied to ER expression positive cases to determine whether the cancer cells are capable of receiving estrogen, concerning the estrogen signal in the breast cancer tissue.
The nature of the cancer cells themselves is important for their proliferation. However, recent findings suggest that the interaction between cancer cells and stromal cells surrounding the cancer cells is also important. Estrogen that has a growth acceleration effect on breast cancer is mainly produced in the ovaries before menopause. Women's blood estrogen concentration is generally rapidly decreased after menopause because of hypoovarianism compared to before menopause. However, locally high levels of estrogen concentration are detected in the breast cancer tissue of postmenopausal women, presumably because estrogen is synthesized by an enzyme called aromatase using androgen as the substrate within the adipose stromal cells surrounding the cancer (referred to as the adipose stromal cell hereafter) in the lacteal gland tissue and released into the lacteal gland tissue.
Recently, particularly for breast cancer after menopause, attempts have been made to treat breast cancer using aromatase inhibitors that inhibit the aromatase activity of the adipose stromal cell and then inhibit the estrogen supply from the stromal cell to the cancer cell so as to suppress the growth of breast cancer. This has been proven to have an effect equal to or more than the drug therapy using tamoxifen that targets the estrogen receptors themselves. The aromatase inhibitor is becoming the first drug option in hormone therapy for postmenopausal breast cancer.
However, it is obviously insufficient for assessing individual applications, namely whether or not some aromatase inhibitor is effective on individual cases, to determine whether or not the estrogen receptors are expressed in the cancer cell, which is conventionally done for drugs targeting the estrogen receptors themselves such as tamoxifen, because the adipose stromal cells surrounding the cancer cells mainly have aromatase enzyme activity. An excellent method of measuring the aromatase enzyme activity and predicting the effectiveness is required.
Some attempts have been made to measure the aromatase enzyme activity in breast cancer tissue. Santer et al. measures the aromatase enzyme activity on the stromal cells of breast cancer or borderline malignant lacteral gland tissue by cultivating the cells in the presence of tritium-labeled androstenedion and measuring released tritium (Journal of Clinical Endocrinology and Metabolism, 82(1), pp 200-208, 2005). Instead of measuring the aromatase enzyme activity itself, Qing Lu et al. measures the expression level of aromatase enzyme protein in the epithelial cell or stromal cell of breast cancer tissue using tissue immunostaining with an antibody to aromatase or western blotting as an alternative to the quantification of the aromatase enzyme activity (Journal of Endocrinology, 137(7), pp 3061-3067., 2005).

### DISCLOSURE OF THE INVENTION

In any case, in order to assess the ability of breast cancer adipose stromal cells to accelerate the growth of breast cancer via aromatase activity, the former method uses RI and therefore is very complicated and the latter method assesses only the aromatase enzyme level, which may not be necessarily correlated to the enzymatic activity. These methods are practically unsatisfactory as a method for clinically assessing the effectiveness of aromatase inhibitors.

As described above, in current hormone therapy, accurate hormonal information is not always grasped before application of the hormone therapy. Clinically, the efficacy is firstly revealed after the drug or the inhibitor is administered. In many cases, the effectiveness of a drug is often specific to respective patients, therefore, a drug may be effective for some patients but it may not be effective for other patients. Once a drug is clinically administered, if the drug is not effective, the period of treatment is prolonged uselessly for that treatment.
Therefore, if a method for cell analysis available for accurate prediction on whether or not the hormone therapy through estrogen signal is effective for an individual patient is provided, useful information can be provided. However, no such cell analysis methods have been known so far.
It is a feature of hormone therapy that side effects of the therapy is generally less serious than chemotherapy. However, the side effects include increased risks of uterine cancer and thrombosis in the patients with prolonged use of tamoxifen and osteoporosis in vase of patients with use of selective aromatase inhibitors. Then, if some information on objective assessment for determining whether or not the hormone therapy is useful for a cancer patient is available in advance, a prescription specific to and useful for the patient can be provided without causing any unnecessary side effects. Such information for objective assessment becomes available if the dynamics of estrogen signal van be grasped at the stage of occurring its activity. However, such information for assessment has not been known so far.

In view of the above circumstances, a purpose of the present invention is to provide an assay system for assessing the estrogen signal in breast cancer tissue, which is significantly important for assessing the pathological condition of breast cancer. More specifically, the first purpose of the present invention is to establish a new transgenic cell line for quantitatively assessing the estrogen signal and the second purpose of the present invention is to provide an assay system for predicting the effectiveness of an aromatase inhibitor (a new drug in the breast cancer hormone therapy) in an individual cancer cases using the same cell line.

The present invention further provides an assay system for predicting the therapeutic effectiveness of an aromatase inhibitor (a new drug in the breast cancer hormone therapy) in individual cancer cases. The present invention further provides a new transgenic cell line for quantitatively assessing the estrogen signal that is significantly important for assessment of the pathological condition of breast cancers and provides an assay system for predicting the therapeutic effectiveness of an aromatase inhibitor (a new drug in the breast cancer hormone therapy) in individual cancer cases using the same cell line.

The present invention further provides a method for cell analysis that enables visualization of intracellular information such as hormonal information.

### 1. Establishment of a new transgenic cell line for quantitatively assessment of the estrogen signal

A new cell line was established for the purpose of quantitatively assessment of the estrogen signal. More specifically, a DNA sequence to which an estrogen receptor activated upon receiving estrogen specifically binds, or ERE (estrogen responsive element), is introduced into a reporter gene in the transcriptional control region to create a reporter gene, which is then stably introduced in a human breast cancer-derived cultured cell strain in which a high level of estrogen receptor is intrinsically expressed to establish a new breast cancer cultured cell line.
As the reporter gene, a green fluorescence protein (GPF) from jellyfish Aequorea is firstly selected. Unlike many other reporter proteins, the GFP expression level can visually be detected and quantified without adding any substrate. Therefore, there is no need of destroying the cell for quantifying the expression level of the reporter protein, then the time-course alteration in the expression level of the reporter protein upon stimulation can be detected in the reporter gene-introduced cell. This characteristic is preferable for the purpose of the present invention, in which case the GFP is selected.
In the present invention, multiple kinds of GFPs having different characteristics were selected and used as the reporter gene to create multiple kinds of reporter genes having an ERE (estrogen responsive element) incorporated in their transcriptional control regions. These reporter genes were introduced in two types of breast cancer cell strains (MCF7 and T47D) in which a high level of estrogen receptor was expressed, and multiple clones in the genome of which the reporter gene was stably introduced were isolated to establish new cell lines. The reaction of the established cell lines toward the estrogen signal was examined.
As a result, the reaction toward the estrogen signal varied among individual kinds of GFPs used as the reporter gene and among the types of the cell lines in which the same reporter gene was introduced. In order to quantitatively assessing the estrogen signal, it was found in the process of the present invention that the nature of GFP or a reporter protein is important and the quantitative assessment is difficult unless the half-life period is approximately 2 hours to 6 hours. Even if the GFP reporter gene having a half-life period of 2 hours to 6 hours, the reaction varies among clones. The cell of the present invention was established by selecting clones in which GFPs were detected dependently on the concentration of added estrogen.

Establishment of an assay system for predicting the effectiveness of an aromatase inhibitor in a breast cancer case
An assay system for predicting the effectiveness of an aromatase inhibitor was established in a breast cancer case, using the cell line as described above. More specifically, an assay system was established by co-cultivating an adipose stromal cell in a breast cancer tissue of a patient and a new breast cancer cultured cell line, wherein the patient is assumed to be producing aromatase and estrogen using androgen as the substrate to increase the local estrogen concentration in the breast cancer tissue thereby largely contributing to the growth of breast cancer, and wherein the cell line is stably introduced with a reporter gene having an ERE (estrogen responsive element) in the transcriptional control region of the reporter gene, and further culturing the cell with the addition of androgen as the substrate for aromatase, whereby the reporter protein is expressed dependently on estrogen signal mainly via aromatase enzymatic activity in the adipose stromal cell, enabling the estrogen signal from the stromal cell to be quantitatively assessed.
Using the above assay, the induction of estrogen signal mainly via aromatase enzyme activity in the adipose stromal cell in the cancer tissue of a patient can be assessed quantitatively. Using the quantitative assessment as an indicator, the ability of the adipose stromal cell to accelerate the cancer cell growth mainly via aromatase activity can be assessed.
Furthermore, the adipose stromal cell and the breast cancer cell line in which the reporter gene is stably introduced are co-cultured and further cultured with the addition of androgen only, which is the substrate for aromatase, (Case 1) and with addition of androgen (testosterone) of the same concentration and an aromatase inhibitor (Case 2). Then, the expression levels of the reporter protein in the reporter gene-introduced breast cancer cells can be compared to quantitatively assess the inhibitory effect of the aromatase inhibitor added. Using the quantitative assessment as an indicator, the antineoplastic activity and effectiveness of the aromatase inhibitor in the cancer case having that adipose stromal cell can be predicted.
In the present invention, it was found in the course of establishing and assessing the assay system that the estrogen signal detected by quantifying the reporter protein is not always correlated to the expression level of the aromatase enzyme gene in the adipose stromal cell. This suggests that some estrogen receptor activation mechanism other than extrinsic estrogen is present, as previously presumed, and that the assay system can also assess the estrogen signal based on the unknown estrogen signal activation mechanism, proving that the assay system is able to quantitatively assess not only the estrogen signal from the aromatase activity in the adipose stromal cell but also the estrogen signal through an unknown estrogen receptor activation mechanism other than estrogen, substantially quantifying the estrogen signal involved in cancer cell growth.

Intensive examination was performed by the inventors of the present invention on co-culture of cell strains from specific tissues in pursue of the above purpose, leading to the method for cell analysis according to the present invention.

The transgenic cell of the present invention is characterized in that a reporter vector containing a gene coding a reporter protein and an estrogen responsive element (ERE) at the upstream of the promoter region of the reporter protein, wherein the expression of the reporter protein is substantially controlled by the estrogen responsive element is stably introduced.

A preferred embodiment of the transgenic cell of the present invention is characterized in that the cell in which a reporter vector is stably introduced is a human breast cancer-derived cultured cell strain.

In a preferred embodiment of the transgenic cell of the present invention is characterized in that the cell in which a reporter vector is stably introduced is a human breast cancer-derived cultured cell strain MCF7 in which a high level of estrogen receptor is intrinsically expressed.

In a preferred embodiment of the transgenic cell of the present invention, it is characterized in that the reporter protein is a protein derived from at least one selected from the group consisting of fluorescent proteins such as GFP and RFP, or from the group consisting of enzymes such as luciferase, β-galactosidase, β-lactamase, and β-glucuronidase.

In a preferred embodiment of the transgenic cell of the present invention, the cell according to any one of Claims 1 to 3 is characterized in that the reporter protein is a protein derived from at least one selected from the group consisting of fluorescent proteins such as GFP and RFP, or from the group consisting of enzymes such as luciferase, β-galactosidase, β-lactamase, and β-glucuronidase, and the half-life period of the protein is approximately 2 hours to 6 hours.

In a preferred embodiment of the transgenic cell of the present invention, it is characterized in that the cell exhibits a difference in the level of reporter gene expression to the extent that it is visualized or distinguished between the estrogen treated case and the estrogen untreated case.

A preferred embodiment of the transgenic cell of the present invention is characterized in that the difference is the difference between the reporter gene expression intensity for the maximum cell number in the estrogen untreated case and the reporter gene expression intensity for the maximum cell number in the estrogen treated case.

A preferred embodiment of the transgenic cell of the present invention is characterized in that with the borderline being set by the reporter gene expression intensity for the maximum cell number in the estrogen treated case, a sufficient number of cells having a high level of expression intensity are present to the extent to achieve visualization by said reporter gene.

A preferred embodiment of the transgenic cell of the present invention is characterized in that when said cell is treated by estrogen, the reporter gene expression level of said cell is changed dependently on the estrogen concentration treated.

A preferred embodiment of the transgenic cell of the present invention is characterized in that the transgenic cell is the cell deposited under accession number FERM BP-10610.

The method for assessing estrogen signal is characterized in that the assessment is achieved by visualizing the estrogen signal response using the cell according to any one of Claims 1 to 10.

The method for quantitatively assessing the induction of estrogen signal of the present invention is a method for quantitatively assessing the induction of estrogen signal mainly via aromatase enzymatic activity derived from a breast cancer tissue-derived adipose stromal cell, the method characterized by comprising the following steps of:
A: co-culturing the cell according to any one of Claims 1 to 10 and a breast cancer tissue-derived adipose stromal cell;
B: adding androgen, which is the substrate for aromatase, to the two co-cultured cells and culturing them;
C: quantifying the reporter protein induced by estrogen that is produced by the adipose stromal cell aromatase and released into the culture solution; and
D: quantitatively assessing the induction of estrogen signal mainly via aromatase enzymatic activity of the adipose stromal cell based on the amount of the reporter protein quantified in the step C.

In a preferred embodiment of the method for quantitatively assessing the induction of estrogen signal of the present invention, it is characterized by that in the step A comprises culturing the cell according to any one of Claims 1 to 10 at the bottom of a culture vessel, adding a breast cancer tissue section obtained by slicing the breast cancer tissue containing adipose stromal cell to the culture vessel using an insert and co-culturing the cell according to any one of Claims 1 to 10 and the breast cancer tissue-derived adipose stromal cell.

The method for quantitatively assessing the induction of estrogen signal of the present invention is a method for quantitatively assessing the induction of estrogen signal mainly via aromatase enzymatic activity that is induced from a breast cancer tissue-derived adipose stromal cell, the method characterized by comprising the following steps of:
A: slicing a breast cancer tissue containing adipose stromal cell in a culture medium and then centrifuging the medium to obtain a supernatant;
B: adding said supernatant and androgen, which is the substrate for aromatase, to the cell according to any one of Claims 1 to 10;
C: quantifying the reporter protein induced by estrogen produced by the adipose stromal cell aromatase and released into the culture solution; and
D: quantitatively assessing the induction of estrogen signal mainly via aromatase enzymatic activity of the adipose stromal cell based on the amount of the reporter protein quantified in said step C.

The method of quantitatively assessing the induction of estrogen signal of the present invention is a method of quantitatively assessing the induction of estrogen signal mainly via aromatase enzyme activity that is induced from a breast cancer tissue-derived adipose stromal cell, characterized by comprising the following steps of:
A: co-culturing the cell according to any one of Claims 1 to 10 and a breast cancer tissue-derived adipose stromal cell;
B: adding androgen (testosterone), which is the substrate for aromatase, to said two co-cultured cells;
C: quantifying the reporter protein induced by both or either one of the estrogen signal induced by estrogen produced by the adipose stromal cell aromatase and released into the culture medium and an estrogen receptor activation mechanism induced from the adipose stromal cell and having no relation with aromatase enzyme; and
D: quantitatively assessing the induction of estrogen signal mainly via aromatase enzymatic activity of the adipose stromal cell based on the amount of the reporter protein quantified in said step C.

A preferred embodiment of the method for quantitatively assessing the inensity of estrogen signal of the present invention is characterized in that said step A comprises culturing the cell according to any one of Claims 1 to 10 at the bottom of a culture vessel, adding a breast cancer tissue section obtained by slicing the breast cancer tissue containing adipose stromal cell to the culture vessel using an insert and co-culturing the cell according to any one of Claims 1 to 10 and the breast cancer tissue-derived adipose stromal cell.

The method for quantitatively assessing the induction of estrogen signal of the present invention is a method for quantitatively assessing the induction of estrogen signal mainly via aromatase enzymatic activity that is derived from a breast cancer tissue-derived adipose stromal cell, the method comprising the following steps of:
A: slicing a breast cancer tissue containing adipose stromal cell in a culture medium and then centrifuging the medium to obtain a supernatant;
B: adding said supernatant and androgen, which is the substrate for aromatase, to the cell according to any one of Claims 1 to 10;
C: quantifying the reporter protein induced by both or either one of the estrogen signal induced by estrogen produced by the adipose stromal cell aromatase and released into the culture medium and an estrogen receptor activation mechanism induced from the adipose stromal cell and having no relation with aromatase enzyme; and
D: quantitatively assessing the induction of estrogen signal mainly via aromatase enzymatic activity of the adipose stromal cell based on the amount of the reporter protein quantified in said step C.

The method for quantitatively assessing the inhibitory effect of the present invention is a method for quantitatively assessing the inhibitory effect of an aromatase inhibitor on estrogen signal mainly via aromatase enzymatic activity that is derived from a breast cancer tissue-derived adipose stromal cell, characterized by comprising the following steps:
A: co-culturing the cell according to any one of Claims 1 to 10 and the breast cancer tissue-derived adipose stromal cell;
B: simultaneously adding androgen, which is the substrate for aromatase, and an aromatase inhibitor to the two co-cultured cells;
C: quantifying the reporter protein induced by estrogen produced by the adipose stromal cell aromatase and released into the culture medium; and
D: quantitatively assessing the inhibitory effect of the aromatase inhibitor on estrogen signal mainly via aromatase enzymatic activity of the adipose stromal cell based on the amount of reporter protein quantified in the step C.

In a preferred embodiment of the method for quantitatively assessing the inhibitory effect of the present invention, it is characterized in that said step A comprises culturing the cell according to any one of Claims 1 to 10 at the bottom of a culture vessel, adding a breast cancer tissue section obtained by slicing the breast cancer tissue containing adipose stromal cell to the culture vessel using an insert and co-culturing the cell according to any one of Claims 1 to 10 and the breast cancer tissue-derived adipose stromal cell.

The method for assessing the inhibitory effect of the invention is a method for quantitatively assessing the inhibitory effect of an aromatase inhibitor on estrogen signal mainly via aromatase enzymatic activity that is derived from a breast cancer tissue-derived adipose stromal cell, the method comprising the following steps of:
A: slicing a breast cancer tissue containing adipose stromal cell in a culture medium and then centrifuging the medium to obtain a supernatant;
B: adding said supernatant and androgen, which is the substrate for aromatase, to the cell according to any one of Claims 1 to 10;
C: quantifying the reporter protein induced by estrogen produced by the adipose stromal cell aromatase and released into the culture medium; and
D: quantitatively assessing the inhibitory effect of the aromatase inhibitor on estrogen signal mainly via aromatase enzymatic activity of the adipose stromal cell based on the amount of the reporter protein quantified in said step C.

The method for analysis of an inhibitor of the present invention is characterized in that an aromatase inhibitor is analyzed as to whether or not it has a tumor suppressive effect specific to a patient by determining the presence/absence of the estrogen signal after the addition of the aromatase inhibitor using the method for assessing according to any one of Claims 18 to 20.

The method for quantitatively assessing the induction of estrogen signal of the present invention is a method for quantitatively assessing the induction of estrogen signal from a breast cancer tissue, the method comprising the following steps of:
A: slicing the breast cancer tissue in a culture medium and then centrifuging the medium to obtain a supernatant;
B: adding the supernatant to the cell according to any one of Claims 1 to 10;
B: quantifying the reporter protein induced by the supernatant; and
C: quantitatively assessing the induction of estrogen signal from the breast cancer tissue based on the amount of the reporter protein quantified in the step C.

The method for quantitatively assessing the induction of estrogen signal of the present invention is a method for quantitatively assessing the induction of estrogen signal from a breast cancer tissue, the method comprising the following steps of:
A: culturing the cell according to any one of Claims 1 to 10 at the bottom of a culture vessel, adding a breast cancer tissue section obtained by slicing the breast cancer tissue to the culture vessel using an insert and co-culturing the cell according to any one of Claims 1 to 10 and said breast cancer tissue section;
B: quantifying the reporter protein induced by said breast cancer tissue section; and
C: quantitatively assessing the induction of estrogen signal from the breast cancer tissue based on the amount of the reporter protein quantified in said step C.

The method for cell analysis of the present invention is characterized in that the method comprising the steps of;
co-culturing a reporter gene-introduced cell in which a gene coding a reporter protein that specifically responses to a chemical substance (A) is introduced and a chemical substance producing cell that takes in a substrate (B) which is a substrate of the reaction for production of the chemical substance (A), expresses an enzyme (C) that brings conversion to the chemical substance (A), and finally produces and releases the chemical substance (A) into the culture medium, and
analyzing the ability of said chemical substance producing cell to produce said chemical substance (A) based on the expression level of the reporter gene from said reporter gene-introduced cell.

A preferred embodiment of the method for cell analysis of the present inventionis is characterized in that the method comprises the steps of; culturing said reporter gene-introduced cell at the bottom of a culture vessel, adding a tissue section obtained by slicing a tissue containing the chemical substance producing cell to the culture vessel using an insert and co-culturing said reporter gene-introduced cell and said chemical substance producing cell.

The method for cell analysis of the present invention is characterized in that the method comprises the steps of;
culturing a reporter gene-introduced cell in which a gene coding a reporter protein that specifically responses to a chemical substance (A) is introduced, with a supernatant obtained by slicing a tissue containing a chemical substance producing cell that takes in a substrate (B) which is a substrate of the reaction for production of the chemical substance (A), expresses an enzyme (C) that brings conversion to the chemical substance (A), and finally produces and releases the chemical substance (A), into a culture medium and centrifuging the medium, and
analyzing the ability of said chemical substance producing cell to produce said chemical substance (A) based on the expression level of the reporter gene from said reporter gene-introduced cell.

A preferred embodiment of the method for cell analysis of the present invention is characterized in that the ability of the chemical substance producing cell to produce the chemical substance (A) is quantitatively analyzed, using the expression level of the reporter gene obtained by culturing the reporter gene-introduced cell in a culture medium containing a known amount (including zero) of the chemical substance (A) in the absence of the chemical substance producing cell, as the comparative reference.

The method for cell analysis of the present invention is characterized in that the method comprising the steps of;
co-culturing a reporter gene-introduced cell in which a gene coding a reporter protein that specifically responses to a chemical substance (A) is introduced and a chemical substance producing cell that takes in a substrate (B) which is a substrate of the reaction for production of the chemical substance (A), expresses an enzyme (C) that brings conversion to the chemical substance (A), and finally produces and releases the chemical substance (A) into the culture medium, in the presence of an inhibitor that inhibits said enzyme (C), and
analyzing the effectiveness of said inhibitor based on the expression level of the reporter gene from said reporter gene-introduced cell.

A preferred embodiment of the method for cell analysis of the present invention is characterized in that the method comprises the steps of; culturing said reporter gene-introduced cell at the bottom of a culture vessel, adding tissue section obtained by slicing a tissue containing said chemical substance producing to the culture vessel using an insert and co-culturing said reporter gene-introduced cell and said chemical substance producing cell in the presence of said inhibitor.

The method for cell analysis of the present invention is characterized in that the method comprises the steps of;
culturing a reporter gene-introduced cell in which a gene coding a reporter protein that specifically responses to a chemical substance (A) is introduced, with a supernatant obtained by slicing a tissue containing a chemical substance producing cell that takes in a substrate (B) which is a substrate of the reaction for production of the chemical substance (A), expresses an enzyme (C) that brings conversion to the chemical substance (A), and finally produces and releases the chemical substance (A), in a culture medium and centrifuging the medium in the presence of an inhibitor that inhibits said enzyme (C), and
analyzing the effectiveness of said inhibitor based on the expression level of the reporter gene from said reporter gene-introduced cell.

A preferred embodiment of the method for cell analysis of the present invention is characterized in that the effectiveness of the inhibitor is quantitatively analyzed, using the expression level of the reporter gene obtained by culturing the reporter gene-introduced cell in a culture medium containing a known amount (including zero) of the chemical substance (A) in the absence of the chemical substance producing cell, as the comparative reference.

A preferred embodiment of the method for cell analysis of the present invention is characterized in that the chemical substance (A) is estrogen, the gene coding a reporter protein is a gene from at least one selected from the group consisting of fluorescent proteins such as GFP and RFP, or from the group consisting of enzymes such as luciferase, β-galactosidase, β-lactamase, and β-glucuronidase, the reporter gene-introduced cell is derived from breast cancer, the substrate (B) is androgen, the enzyme (C) is aromatase, and the chemical substance producing cell is an adipose stromal cell isolated from a breast cancer tissue.

A preferred embodiment of the method for cell analysis of the present invention is characterized in that the chemical substance (A) is estrogen, the gene coding a reporter protein is a gene from at least one selected from the group consisting of fluorescent proteins such as GFP and RFP, or from the group consisting of enzymes such as luciferase, β-galactosidase, β-lactamase, and β-glucuronidase and the reporter protein coded by the gene has a half-life period of approximately 2 hours to 6 hours, the reporter gene-introduced cell is derived from breast cancer, the substrate (B) is androgen, the enzyme (C) is aromatase, and the chemical substance producing cell is an adipose stromal cell isolated from a breast cancer tissue.

A preferred embodiment of the method for cell analysis of the present invention is characterized in that the chemical substance (A) is estrogen, the reporter gene-introduced cell is the transgenic cell according to any one of Claims 1 to 10, the substrate (B) is androgen, the enzyme (C) is aromatase, and the chemical substance producing cell is an adipose stromal cell isolated from a breast cancer tissue.

A preferred embodiment of the method for cell analysis of the present invention is characterized in that the analysis is conducted based on the average value of the expression level of the reporter gene.

A preferred embodiment of the method for cell analysis of the present invention is characterized in that the expression level of the reporter gene is measured based on an acquired image.

A preferred embodiment of the method for cell analysis of the present invention is characterized in that said reporter gene-introduced cell is cultured to monolayer at the bottom of a culture vessel, said chemical substance producing cell is co-cultured to monolayer on a base having a culture surface at a distance L above said culture vessel bottom, and an image of said reporter gene-introduced cell is acquired from downward of said culture vessel via an objective lens.

A preferred embodiment of the method for cell analysis of the present invention is characterized in that the base is detachable and the image is acquired after detachment of the base.

A preferred embodiment of the method for cell analysis of the present invention is characterized in that the distance L is not within the range of the focal length of the objective lens and the image is acquired without detachment of the base.

A preferred embodiment of the method for cell analysis of the present invention is characterized in that the nucleus of the reporter gene-introduced cell is labeled with a spectral property different from that of the introduced reporter protein and the reporter gene-introduced cell is identified by a fluorescent or luminous image of the nuclei through image processing to assess the expression level of the reporter protein.

A preferred embodiment of the method for cell analysis of the present invention, is characterized in that the luminance property of a nuclei image is processed to identify the cell in the image, the nucleus region identified through image processing is binary-processed, the fluoresce or luminescence level in the area corresponding to the obtained binary image is measured for each cell to assess the expression level of the reporter gene.

A preferred embodiment of the method for cell analysis of the present invention is characterized in that the nucleus is labeled with a wavelength longer than the spectral property of the reporter protein.

A preferred embodiment of the method for cell analysis of the present invention is characterized in that the reporter protein is a green fluorescence protein.

A preferred embodiment of the method for cell analysis of the present invention is characterized in that the nucleus is labeled with DRAQ5.

A preferred embodiment of the method for cell analysis of the present invention is characterized in that the amount (including zero) of the chemical substance (A) or/and the inhibitor to be analyzed is previously quantified and attached inside of the wells of a microplate at the solid state and said chemical substance (A) or/and said inhibitor is melted with a given solvent for the analysis.

A preferred embodiment of the method for cell analysis of the present invention is characterized in that t the expression level of the reporter gene is measured within 24 to 96 hours after the beginning of the co-culture.

According to the present invention, the property of the breast cancer can advantageously be analyzed by co-culturing the transgenic cell of the present invention and a breast cancer tissue-derived stromal cell or a breast cancer tissue containing the cell as an estrogen signal indicator cell or by culturing the transgenic cell with the addition of a breast cancer tissue extract.

The method for cell analysis of the present invention can advantageously provide a method for cell analysis for visualizing intracellular signal transduction such as hormonal information in advance.

### BRIEF DESCRIPTION OF THE DRAWINGS

[Fig.1] Fig.1 is an illustration of a constructed estrogen signal reporter gene ERE-GFP.
[Fig.2] Fig.2 shows GFP expression assessment (fluorescence microscopic assessment) of MCF7 cell lines in which the established ERE-GFP is stably introduced (ERE-GFP-E series) and a MCF7 cell line in which only a pd2EGFP-1 vector having no ERE is introduced with/without E₂ treatment.
[Fig.3] Fig.3 shows GFP expression assessment (flow cytometer assessment) of MCF7 cell lines in which the established ERE-GFP is stably introduced (ERE-GFP-E series) and a MCF7 cell line in which only a pd2EGFP-1 vector having no ERE is introduced with/without E₂ treatment.
[Fig.4] Fig.4 shows GFP expression assessment of the ERE-GFP-E10 cell line that is induced with the addition of E₂ and result of counting the living cells.
[Fig.5] Fig.5 shows detection of estrogen signal from breast cancer by co-culturing a breast cancer tissue-derived adipose stromal cell and the MCF7 cell line in which the ERE-GFP is stably introduced ERE-GFP-E10.
[Fig.6] Fig.6 shows detection of estrogen signal in breast cancer cases by co-culturing a breast cancer tissue-derived adipose stromal cell and the MCF7 cell line in which the ERE-GFP is stably introduced ERE-GFP-E10.
[Fig.7] Fig.7 shows detection of estrogen signal in breast cancer cases by co-culturing a breast cancer tissue-derived adipose stromal cell and the MCF7 cell line in which the ERE-GFP is stably introduced ERE-GFP-E10.
[Fig.8] Fig.8 shows quantification of estrogen signal in breast cancer cases by co-culturing a breast cancer tissue-derived adipose stromal cell and the MCF7 cell line in which the ERE-GFP is stably introduced ERE-GFP-E10 and the expression level of aromatase gene mRNA in the adipose stromal cell.
[Fig.9] Fig.9 shows analysis of inhibitory effect of various aromatase inhibitors on estrogen signal by co-culturing a breast cancer tissue-derived adipose stromal cell and the MCF7 cell line in which the ERE-GFP is stably introduced ERE-GFP-E10 in the presence of the aromatase inhibitors.
[Fig. 10] Fig. 10 shows quantitative detection of estrogen signal induction by breast cancer tissue using the supernatants extracted from surgically removed breast cancer tissue specimens.
[Fig.11] Fig.11 sows quantitative detection of estrogen signal induction by breast cancer tissue by co-culturing with a surgically removed and a sliced breast cancer tissue specimen using an insert well.
[Fig. 12] Fig. 12 shows a microplate having 24 wells.
[Fig. 13] Fig. 13 is a cross-sectional view of the microplate.
[Fig.14] Fig.14 is a flowchart showing the process of cell image analysis.
[Fig. 15] Fig. 15 is a flowchart showing the process of image analysis.
[Fig.16] Fig. 16 is an illustration showing the cell area division in a binary image.
[Fig.17] Fig.17 is an illustration showing the identification of multiple cell nuclei.

### BEST MODE FOR CARRYING OUT THE INVENTION

The present invention is further explained in detail hereafter.

A reporter vector containing a gene coding a reporter protein and an estrogen responsive element (ERE) at the upstream of the promoter region of the reporter protein, wherein the expression of the reporter protein is substantially controlled by the estrogen responsive element is stably introduced in the transgenic cell of the present invention.
In preparing the cell of the present invention, the cells utilized as the host cell in which a DNA is introduced include human breast cancer-derived cultured cells. Such cells include human MCF7 and T47D cells.
Here, the estrogen recognition sequence means a specific base sequence generally called the estrogen responsive element (ERE) that is present in the transcriptional control region of a target gene of which the transcription activity is controlled by estrogen receptor. The estrogen-estrogen receptor complex recognizes and binds to the sequence, thereby estrogen-dependently accelerating the transcription of the target gene. Such estrogen recognition sequences specifically include PS2 ERE, progesterone receptor, vitellogenin that is an egg yolk protein precursor, and egg white ovalbumin ERE. The DNA having such a sequence can be chemically synthesized or cloned by PCR. A DNA consisting of a basic sequence containing one or more of the ERE consensus sequence [5'-AGGTCAnnnTGACCTT-3' (SEQ. ID. No.1)] can be chemically synthesized. The promoter sequence can be, for example, TK (thymidine kinase).

In preparing the cell of the present invention, the DNA used for transfection of a host cell (cultured cell) can contain a marker gene for cell selection that is functional within the host cell in addition to the reporter gene. Here, the reporter gene means a gene cording a reporter protein. The marker gene for cell selection means a gene coding an expressive trait that can be a marker for distinguishing the cell transfected with a DNA containing the gene from non-tranfected cells. Being functional within the host cell means that the trait is expressed within the cultured cell. An example of such gene is a gene that is under control of the promoter capable of initiating transcription in the cultured cell, competent for expression in the cultured cell, and codes an expressive trait for effective cell selection in the cultured cell. Selection marker genes effective in the cultured cell include genes imparting the cell resistance to drugs suppressing or inhibiting the growth of the cultured cell. Their specific examples include neomycin resistance-imparting genes (aminoglycosidephosphotransferase genes), hygromycin resistance-imparting genes (hygromycinphosphotransferase genes), and blasticidin S resistance-imparting genes (blasticidin S deminase genes). Blasticidin S resistance-imparting genes are preferable because the trandfected cell can be selected in a short term. Blasticidin S resistance-imparting genes can be obtained for example from a commercially available plasmid pUCSV-BSD.

"A DNA containing a gene coding a reporter protein and an estrogen responsive element (ERE) at the upstream of the promoter region of the reporter protein" can be prepared for example by incorporating these genes in a vector. The vector desirably has a compact size for easy handling and reduced risks of missing from the chromosome of vector inter-molecular or intra-molecular gene recombination or stable transgenic cells. It can be for example approximately 2 kb to 10 kb plasmids. Using E.coli as the host cell for introducing the genes in the vector makes the process efficient. Therefore, it is preferable to have functions as an E.coli vector such as replication initiation site functional in the E.coli, drug-resistant genes, and restriction enzyme recognition sites for inserting the genes.
More specifically, a DNA consisting of a nucleotide sequence containing an estrogen receptor recognition sequence derived from the 5'-upstream region of a Xenopus laevis vitellogenin gene and a minimum promoter from a mouse metallothionein I gene are introduced in a plasmid having a firefly luciferases gene (reporter gene) upstream of this gene. A blasticidin S resistance-imparting gene coupled for example to a SV40 initial promoter is incorporated in the plasmid to prepare a DNA having the above structure.

In a preferred embodiment of the transgenic cell of the present application, the reporter protein is a protein derived from at least one selected from the group consisting of fluorescent proteins such as GFP and RFP, or from the group consisting of enzymes such as luciferase, β-galactosidase, β-lactamase, and β-glucuronidase. The reporter protein is preferably a fluorescent protein such as GFP and RFP. In a preferred embodiment, the half-life period of the protein is approximately 2 hours to 6 hours.

With the gene being introduced, the expression level of a substance expressed upon binding of the estrogen-estrogen receptor complex to the estrogen recognition sequence thereof can be visualized. Here, the expressed substance includes elements involving the growth of cancer.

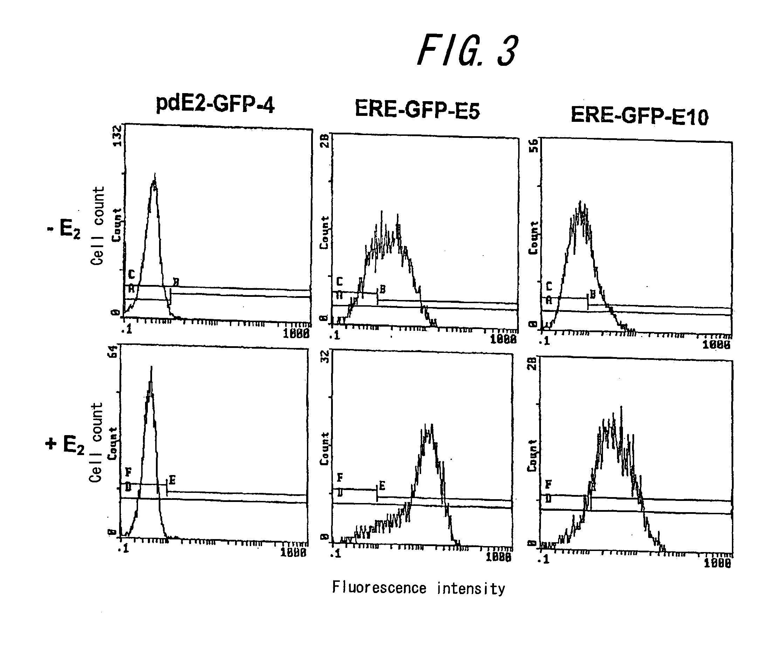

In a preferred embodiment of the transgenic cell of the present application, the cell exhibits a difference in the reporter gene expression level to the extent that it is visualized or distinguished between the estrogen treated case and the estrogen untreated case. This is explained in detail with reference to Fig.3. Fig.3 shows GFP expression assessment #2 - flow cytometer assessment of established MCF7 cell lines in which the ERE-GFP is stably introduced (ERE-GFP-E series) and a MCF7 cell line in which only a pd2EGFP-1 vector having no ERE is introduced with/without E₂ treatment.

In Fig.3, the pdE2-GFP-4 is the control. The pdE2-GFP-4 had no ERE (estrogen recognition sequence). The ERE-GFP-E5 and E10 are the cell of the present invention. Fig.3 shows the relationship between the cell count and the fluorescence intensity from the reporter gene with no addition of estrogen (-E2 at the top) and with the addition of estrogen (+E2 at the bottom).

The estrogen-treated case means the case +E2 and the estrogen-untreated case means the case -E2 in Fig.3. The expression level of a substance expressed upon binding to the estrogen recognition sequence being different between the two cases to the extent that visualization by the reporter gene is available means that there is a difference in the fluorescence intensity for the maximum cell count between the cases -E2 and +E2 in Fig.3. It is preferable that the difference in the fluorescence intensity is sufficiently large for increased detection sensitivity. It is also preferable that the peak determined by the cell count and fluorescent intensity is sharper for increased detection sensitivity. In this respect, it is understood from the comparison between E5 and E10 in Fig.3 that E10 has a sharper form.

When estrogen is added (+E2), it is preferable that the difference in the fluorescence intensity is sufficiently apparent compared to when no estrogen is added. In the viewpoint of increased detection sensitivity, it is preferable that the fluorescence intensity is lower when no estrogen is added. In this respect, it is understood from the comparison between E5 and E 10 in Fig.3 that E10 exhibited lower fluorescence_intensities when no estrogen is added and the difference in the fluorescence intensity is larger between the +E2 case and the no estrogen case.

In a preferred embodiment of the transgenic cell of the present application, with the borderline being set by the reporter gene expression intensity for the maximum cell number in the estrogen treated case, a sufficient number of cells having a high level of expression intensity are present to the extent that visualization by the reporter gene is available.
Here, the comparison is made between E5 and E10 in the case +E2 in Fig.3. In E5, the expression intensity at the position of the expression intensity for the maximum cell count in the case -E2 remained in the case +E2; however, it did not in E10. The expression intensity is nearly horizontally symmetric about the expression intensity for the maximum cell count and the cell count is larger for intensities larger than the expression intensity for the maximum cell count, which is obviously distinguishable from the expression intensity of the case -E2. Therefore, when E5 and E10 are compared, E10 is a cell having a better detection sensitivity and a better quality.

The above described E10 is deposited at the Patent Organism Depositary, National Institute of Advanced Industrial Science and Technology (Tsukuba Central 6, 1-1-1 Higashi, Tsukuba, Ibaraki, Japan) under accession number of FERM BP-10610 as of May 25, 2005.

A method of preparing the cell of the present invention is described hereafter.
In order to prepare the cell of the present invention, "a DNA containing a gene coding a reporter protein and an estrogen responsive element (ERE) at the upstream of the promoter region of the reporter protein" as described above is introduced in a cultured cell and a stable transgenic cell is selected. More specifically, a host cell such as an MCF7 cell is seeded in a Petri dish (10⁵ to 10⁷ cells/Petri dish of 6 to 10 cm in diameter) and cultured with an αMEM medium containing approximately 5 to 10% serum at approximately 37 °C and saturated humidity with 5% CO₂ for several hours to overnight. Then, the DNA is introduced into the cultured cell. The DNA is introduced by a conventional technique such as electroporation, calcium phosphate, and lipofection techniques. The DNA introduced in the host cell desirably has the equal or nearly equal purity to the plasmid DNA purified by CsCl density-gradient centrifugation. As for the form of the DNA introduced in the host cell, a circular plasmid DNA in which the reporter gene and cell selection maker gene are incorporated can be introduced in the host cell as it is. However, it is generally preferable that a linear DNA obtained by cutting at a restriction enzyme site in the region giving no influence on any gene expression is introduced in the host cell. For obtaining a cell that is stably transformed by the introduced DNA, for example, the cell in which the DNA is introduced as described above is cultured in a conventional cell culture solution (medium) for approximately one day. Then, the cell is exfoliated by a routine technique (trypsin treatment) and reseeded and immediately cultured under selection conditions corresponding to the cell selection maker gene introduced in the host cell. In other words, when the cell selection maker gene is a gene that renders drug resistance, a drug to which the transformed cell is made resistant is added to the medium and the cell is cultured until the colony from the transformed cell has a proper size. Meanwhile, if necessary, the medium is replaced with a fresh medium containing the drug one to three times per week. The colony obtained in this way is divided into multiple parts and reseeded to grow the cell. Some of them are cultured with the addition of a solution of a targeted estrogen receptor ligand in a solvent for 24 hours and the expression level of the reporter gene is measured. As control, the expression level in the system to which only the solvent is added is measured. The measurement of the expression level of the reporter gene depends on the types of reporter genes. Except for some reporter gene secreting the product in the medium, the expression level of the reporter gene is measured by cell solvent treatment or by destroying the cell membrane of the cell by ultrasonic treatment to prepare a cell extract solution and quantifying the reporter gene product contained in the extract solution. For example, when the reporter gene product is an enzyme protein, the enzyme protein in the extract solution is reacted with a substrate specific to the enzyme. The produced luminescence, fluorescence, or absorbance is measured to quantify the enzyme activity from the reporter gene product as an indicator of the reporter gene product and eventually the reporter gene expression level. Cells having at least two times and preferably five times higher expression levels of the reporter gene in the system in which the cell and ligand were contacted than in the system in which only the solvent is added are selected. When the cell obtained in this way does not consist of a single transfected cell, the cell is cultured in the limiting dilution procedure and colonies consisting of a single cell can be selected.

The method for assessing the estrogen signal of the present invention is described hereafter. The method for assessing the estrogen signal of the present invention uses the above described cell of the present invention to visualize the estrogen signal response for assessing.

First, the principle of the method for assessing the estrogen signal of the present invention is described. In the cell of the present invention, when a transcription factor binding to the estrogen recognition sequence binds the estrogen recognition sequence, the downstream gene is expressed upon the binding. The downstream gene is presumably a factor to grow cancer cells. In the cell of the present invention, the gene downstream of the estrogen recognition sequence is expressed and an incorporated reporter gene is expressed. Therefore, the expression of the downstream gene can be visualized by measuring the expression level of the reporter gene. In other words, the estrogen signal is assessed. The method for assessing of the present invention uses this principle.

A typical transcription factor biding to the estrogen recognition sequence is an estrogen-estrogen receptor complex. However, research by the inventors of the present invention through the present invention has suggested the involvement of other factors.

The method for quantitatively assessing the induction of estrogen signal of the present invention is a method for quantitatively assessing the induction of estrogen signal mainly via aromatase enzymatic activity derived from a breast cancer tissue-derived adipose stromal cell, the method comprising the following steps of:
A: co-culturing the cell according to any one of Claims 1 to 10 and a breast cancer tissue -derived adipose stromal cell;
B: adding androgen, which is the substrate for aromatase, to the two co-cultured cells and culturing them;
C: quantifying the reporter protein induced by estrogen produced by the adipose stromal cell aromatase and released into the culture medium; and
D: quantitatively assessing the induction of estrogen signal mainly via aromatase enzymatic activity of the adipose stromal cell based on the amount of the reporter protein quantified in the step C.
With this method, the estrogen signal induced by adipose stromal cell via the estrogen production reaction by the aromatase enzyme can be measured quantitatively. Furthermore, different signals can be found in individual cancer cases from the result of the estrogen signal assessment. The result of the assessment is expected to be applicable to a cancer case-specific treatment
As for the transgenic cell of the present invention, the above explanation is applicable as it is.

In the method for assessing, it is preferable that the step A comprises culturing the cell according to any one of Claims 1 to 10 at the bottom of a culture vessel, adding a breast cancer tissue section obtained by slicing the breast cancer tissue containing adipose stromal cell to the culture vessel using an insert and co-culturing the cell according to any one of Claims 1 to 10 and the breast cancer tissue-derived adipose stromal cell. By co-culturing the transgenic cell of the present invention and a breast cancer tissue-derived adipose stromal cell as described above, the expression induction of the reporter gene within the transgenic cell of the present invention can be measured in an in vivo-like environment with accuracy. The insert can be an insert having a porous membrane generally used for cell culture.

In the viewpoint of simplifying the operation and quickly assessing the estrogen signal, it is possible that an adipose stromal cell-containing breast cancer tissue is sliced in a culture medium, then centrifuged to obtain a supernatant, and the supernatant, androgen which is the substrate for aromatase, and an aromatase inhibitor are added to the cell according to any one of Claims 1 to 10 for culture, instated of co-culturing the cell according to any of Claims 1 to 10 and a breast cancer tissue-derived adipose stromal cell and adding androgen to the two co-cultured cells for culture.

The method for quantitatively assessing the induction of estrogen signal of the present invention is a method for quantitatively assessing the induction of estrogen signal mainly via aromatase enzymatic activity that is induced from a breast cancer tissue-derived adipose stromal cell, the method comprising the following steps of:
A: co-culturing the cell according to any one of Claims 1 to 10 and a breast cancer tissue-derived adipose stromal cell;
B: adding androgen, which is the substrate for aromatase, to the two co-cultured cells;
C: quantifying the reporter protein induced by estrogen induced by estrogen produced by the adipose stromal cell aromatase and released into the culture medium and by an estrogen receptor activation mechanism induced from the adipose stromal cell and having no relation with aromatase enzyme; and
D: quantitatively assessing the induction of estrogen signal mainly via aromatase enzymatic activity of the adipose stromal cell based on the amount of the reporter protein quantified in the step C.

Also in this method for assessing, similarly to the above described method for assessing, from the viewpoint that the expression level of the reporter gene within the transgenic cell of the present invention can be measured in an in vivo-like environment with accuracy, it is preferable that the step A comprises culturing the cell according to any one of Claims 1 to 10 at the bottom of a culture vessel, adding a breast cancer tissue section obtained by slicing the breast cancer tissue containing adipose stromal cell to the culture vessel using an insert and co-culturing the cell according to any one of Claims 1 to 10 and the breast cancer tissue-derived adipose stromal cell. Furthermore, similarly to the above described method for assessing, in the viewpoint of simplifying the operation and quickly assessing the estrogen signal, it is possible that an adipose stromal cell-containing breast cancer tissue is sliced in a culture medium, then centrifuged to obtain a supernatant, and the supernatant, androgen which is the substrate for aromatase, and an aromatase inhibitor are added to the cell according to any one of Claims 1 to 10 for culture, instated of co-culturing the cell according to any of Claims 1 to 10 and a breast cancer tissue-derived adipose stromal cell and adding androgen to the two co-cultured cells for culture.

The method for quantitatively assessing the induction of estrogen signal of the present invention is a method for quantitatively assessing the inhibitory effect of an aromatase inhibitor on estrogen signal mainly via aromatase enzymatic activity that is derived from a breast cancer tissue-derived adipose stromal cell, the method comprising the following steps of:
A: co-culturing the cell according to any one of Claims 1 to 8 and a breast cancer tissue-derived adipose stromal cell;
B: simultaneously adding androgen, which is the substrate for aromatase, and an aromatase inhibitor to the two co-cultured cells;
C: quantifying the reporter protein induced by estrogen produced by the adipose stromal cell aromatase and released into the culture medium; and
D: quantitatively assessing the inhibitory effect of the aromatase inhibitor on estrogen signal mainly via aromatase enzymatic activity of the adipose stromal cell based on the amount of reporter protein quantified in the step C.
With this method for assessing, the estrogen signal suppressing effect of various aromatase inhibitors in individual cancer cases can quantitatively assessed. The sensitivity to hormone therapies using various aromatase inhibitors can be assessed in individual cases.

Also in this method for assessing, similarly to the above described two methods for assessing, from the viewpoint that the expression level of the reporter gene within the transgenic cell of the present invention can be measured in an in vivo-like environment with accuracy, it is preferable that the step A comprises culturing the cell according to any one of Claims 1 to 10 at the bottom of a culture vessel, adding a breast cancer tissue section obtained by slicing the breast cancer tissue containing adipose stromal cell to the culture vessel using an insert and co-culturing the cell according to any one of Claims 1 to 10 and the breast cancer tissue-derived adipose stromal cell. Furthermore, similarly to the above described two assessment methods, in the viewpoint of simplifying the operation and quickly assessing the estrogen signal, it is possible that an adipose stromal cell-containing breast cancer tissue is sliced in a culture medium, then centrifuged to obtain a supernatant, and the supernatant, androgen which is the substrate for aromatase, and an aromatase inhibitor are added to the cell according to any one of Claims 1 to 10 for culture, instated of co-culturing the cell according to any of Claims 1 to 10 and a breast cancer tissue-derived adipose stromal cell and adding androgen to the two co-cultured cells for culture.

Here, aromatase is an enzyme present in peripheral adipose tissues and the like. Androgen produced in the adrenal cortex is transformed into estrogen by aromatase. Aromatase inhibitors inhibit the synthesis of estrogen from androgen. Therefore, the hormone therapy using aromatase inhibitors to suppress the growth of breast cancer is extensively used.

If the expression level of the reporter gene is reduced by adding an aromatase inhibitor to the cell of the present invention, the presence/absence of expression of a gene involved in the growth of cancer downstream of the estrogen recognition sequence can be assessed. It is because the expression of the gene involved in the growth of cancer and the expression of the reporter gene of the cell of the present invention are synchronized.

Therefore, a patient-specific aromatase inhibitor that suppresses the expression of the cancer cell growth factor can quickly be found by estrogen signal assessment of the present invention.

In the method for analysis of the inhibitor of the present invention, an aromatase inhibitor is analyzed as to whether or not it has a tumor suppression effect specific to a patient by determining the presence/absence of estrogen signal after the addition of the aromatase inhibitor using the estrogen signal assessment method of the present invention.

If there is any estrogen signal, in other words, the reporter gene is expressed, the aromatase inhibitor did not work. On the other hand, if there is no estrogen signal, in other words, the reporter gene was not expressed, the aromatase inhibitor presumably worked and the aromatase inhibitor is found to be an inhibitor specifically effective to that cancer patient.

The method for quantitatively assessing the induction of estrogen signal of the present invention is a method for quantitatively assessing the induction of estrogen signal from a breast cancer tissue, the method comprising the following steps of:
A: slicing a breast cancer tissue in a culture medium and then centrifuging the medium to obtain a supernatant;
B: adding the supernatant to the cell according to any one of Claims 1 to 10;
B: quantifying the reporter protein induced by the supernatant; and
C: quantitatively assessing the induction of estrogen signal from the breast cancer tissue based on the amount of the reporter protein quantified in the step C.

The estrogen receptor is known for being activated through phosphorylation by gwoth factors such as EGF and IGF-1. The production of these growth factors is largely influenced by microscopic breast cancer environments including the adipose stromal cell. In this method for assesing, the control of the estrogen signal by the microscopic breast cancer environments is comprehended and the overall estrogen signal can be comprehended. Furthermore, the growth effect on the breast cancer cell can also be quantified. Factors other than estrogen are also effectively analyzed. The estrogen receptor activation capability and breast cancer growth acceleration effect can be comprehended in individual cases in a simple and prompt manner. It is applicable to prompt prediction of the effectiveness of hormone therapy.

The method for quantitatively assessing the induction of estrogen signal of the present invention is a method for quantitatively assessing the induction of estrogen signal from a breast cancer tissue, the method comprising the following steps of:
A: culturing the cell according to any one of Claims 1 to 10 at the bottom of a culture vessel, adding a breast cancer tissue section obtained by slicing the breast cancer tissue to the culture vessel using an insert and co-culturing the cell according to any one of Claims 1 to 10 and the breast cancer tissue section;
B: quantifying the reporter protein induced by the breast cancer tissue section; and
C: quantitatively assessing the induction of estrogen signal from the breast cancer tissue based on the amount of the reporter protein quantified in the step C.
With this method for assessing, the overall estrogen signal can immediately be comprehended in a more in vivo-like environment.

The method for cell analysis of the present invention is described hereafter. In the method for cell analysis of the present invention, a reporter gene-introduced cell in which a gene coding a reporter protein that specifically responses to a chemical substance (A) is introduced and a chemical substance producing cell that takes in a substrate (B) which is a substrate of the reaction for production of the chemical substance (A), expresses an enzyme (C) that brings conversion to the chemical substance (A), and finally produces and releases the chemical substance (A) into the culture medium are co-cultured and the ability of the chemical substance producing cell to produce the chemical substance (A) is analyzed based on the expression level of the reporter gene from the reporter gene-introduced cell. The reporter gene-introduced cell in which a reporter gene specifically responses to a chemical substance (A) is introduced means a transgenic cell in which a reporter gene is incorporated in the manner that the presence of a chemical substance (A) finally causes the reporter gene to be expressed after various cascade reactions in sync with genes controlled by that transcription factor as the transcription factor.
With the above method, the status of expression of an operon, transcription of which is controlled by the presence of the chemical substance (A), can be visualized via the reporter gene.
The reported-gene introduced cell can be those obtained by introducing in the chromosome of a cancer cell-derived cultured cell a DNA containing a cell selection marker gene that can be functional together with the reporter gene within the cultured cell.
For example, when the transgenic cell is a breast cancer-derived cell, a reporter vector containing a gene coding a reporter protein and an estrogen responsive element (ERE) at the upstream of the promoter region of the reporter protein, wherein the expression of the reporter protein is substantially controlled by the estrogen responsive element is stably introduced in the transgenic cell as in the above described transgenic cell of the present invention. The above explanation is applicable to the transgenic cell of the present invention as it is.
The method for cell analysis of the present invention co-cultures the reporter gene-introduced cell as exemplified above and a chemical substance producing cell. The chemical substance producing cell is a cell that takes in a substrate (B) which is a substrate of the reaction for production of the chemical substance (A), expresses an enzyme (C) for conversion to the chemical substance (A), and finally produces and releases the chemical substance (A) into the culture medium. By co-culturing the reporter gene-introduced cell and chemical substance producing cell, the interaction between the chemical substance producing cell providing a chemical substance and the cell having a function to response to the chemical substance can be visualized through the reporter gene.

In a preferred embodiment, from the viewpoint of measuring the expression intensity of the reporter gene in the reporter gene-introduced cell in an in vivo-like environment with accuracy, it is possible that the reporter gene-introduced cell is cultured at the bottom of a culture vessel and a tissue section obtained by slicing a tissue containing the chemical substance producing cell is added to the culture vessel using an insert to co-culture the reporter gene-introduced cell and the chemical substance producing cell.

In a preferred embodiment, from the viewpoint of simplifying the operation and quickly assessing estrogen signal, the reporter gene-introduced cell can be cultured with a supernatant obtained by slicing a tissue containing a chemical substance producing cell and centrifuging it, instead of co-culturing the reporter gene-introduced cell and the chemical substance producing cell.

For example, In a preferred embodiment, the ability of the chemical substance producing cell to produce the chemical substance (A) can be quantitatively analyzed, using the expression level of the reporter gene obtained by culturing the reporter gene-introduced cell in a culture medium containing a known amount (including zero) of the chemical substance (A) in the absence of the chemical substance producing cell, as the comparative reference.

In another embodiment of the method for cell analysis of the present invention, it is possible that a reporter gene-introduced cell in which a gene coding a reporter protein specifically responses to a chemical substance (A) is introduced and a chemical substance producing cell that takes in a substrate (B) which is a substrate of the reaction for production of the chemical substance (A), expresses an enzyme (C) that brings conversion to the chemical substance (A), and finally produces and releases the chemical substance (A) into the culture medium are co-cultured in the presence of an inhibitor inhibiting the enzyme (C), and the effectiveness of the inhibitor is analyzed based on the expression level of the reporter gene from the reporter gene-introduced cell.

In a preferred embodiment, from the viewpoint of measuring the expression intensity of the reporter gene in the reporter gene-introduced cell in an in vivo-like environment with accuracy, it is possible that the reporter gene-introduced cell is cultured at the bottom of a culture vessel and a tissue section obtained by slicing a tissue containing the chemical substance producing cell is added to the culture vessel using an insert to co-culture the reporter gene-introduced cell and the chemical substance producing cell in the presence of the inhibitor.

In a preferred embodiment, from the viewpoint of simplifying the operation and quickly assessing estrogen signal, the reporter gene-introduced cell can be cultured with a supernatant obtained by slicing a tissue containing the chemical substance producing cell and centrifuging it in the presence of the inhibitor, instead of co-culturing the reporter gene-introduced cell and the chemical producing cell in the presence of the inhibitor.

In a preferred embodiment, the effectiveness of the inhibitor can quantitatively be analyzed, using the expression level of the reporter gene obtained by culturing the reporter gene-introduced cell in a culture medium containing a known amount (including zero) of the chemical substance (A) in the absence of the chemical producing cell, as the comparative reference.

In the above method for cell analysis of the present invention, preferably, the chemical substance (A) is estrogen, the gene coding a reporter protein is a gene from at least one selected from the group consisting of fluorescent proteins such as GFP and RFP, or from the group consisting of enzymes such as luciferase, β-galactosidase, β-lactamase, and β-glucuronidase, the reporter gene-introduced cell is derived from breast cancer, the substrate (B) is androgen, the enzyme (C) is aromatase, and the chemical substance producing cell is an adipose stromal cell isolated from a breast cancer tissue. More preferably, the gene coding a reporter protein is a gene from at least one selected from the group consisting of fluorescent proteins such as GFP and RFP, or from the group consisting of enzymes such as luciferase, β-galactosidase, β-lactamase, and β-glucuronidase and the reporter protein coded by the gene has a half-life period of approximately 2 hours to 6 hours. Further preferably, the reporter gene-introduced cell is the above described transgenic cell of the present invention. Because of excellent detection sensitivity, the cell deposited with the accession number BP-10610 is most preferably used as the reporter gene-introduced cell in the method for cell analysis of the present invention.

In a preferred embodiment, it is possible that the analysis is conducted based on the average value of the expression level of the reporter gene and the expression level of the reporter gene is measured based on an acquired image.

The ability of the stromal cell to produce aromatase can quantitatively be analyzed by culturing the reporter gene-introduced cell in a culture medium with the addition of a given amount (including zero) of estrogen and measuring and comparing the fluorescence or luminescence from the reporter gene-introduced cell. In this way, the influence of estrogen on the ability to produce the chemical substance is visualized and known. The effect of an aromatase inhibitor on the cultured cell line can be visualized. For example, the most effective aromatase inhibitor for a patient-specific cancer cell can be examined before it is administered to the patient.

In another embodiment, the reporter gene-introduced cell is cultured to monolayer at the bottom of a culture vessel, the chemical substance producing cell is co-cultured to monolayer on a base having a culture surface at a distance L above the culture vessel bottom, and an image of the reporter gene-introduced cell is acquired from downward of the culture vessel via an objective lens. The reason for the distance L is that the self-fluorescence or self-luminescence from the chemical substance producing cell and reflected light from illumination source become background light and reduces the signal light S/N from the reporter gene-introduced cell. The distance L preferably is at least not within the range the focal length of the objective lens because a sufficiently high S/N of the signal light from the reporter gene-introduced cell should be maintained. In such a case, fluorescent or luminescent images are captured without detachment of the base.

In a preferred embodiment, the base is detachable and the fluorescent or luminescent image is acquired after detachment of the base. The detachable base facilitates the analysis; the fluorescence or luminescence can be measured in a stable manner with no loss in the signal light S/N from the reporter gene-introduced cell.

When the reporter gene is derived from a fluorescent protein, the fluorescent protein to be introduced preferably has a half-life period of less than four hours.

In order to measure the signal light from the reporter gene-introduced cell with accuracy, it is important to precisely identify the reporter gene-introduced cell using image processing techniques. Generally, there are many fluorescent substances such as dead cells and contaminants in the cell culturing environments. If they are detected as the signal light, the accuracy of analysis is significantly deteriorated. In order to identify the reporter gene-introduced cell by image processing, the reporter gene-introduced cell can easily be identified in the image processing by labeling the nuclei apart from the light from the reporter gene. This is because the nuclei do not share the borders with adjacent cells unlike the whole cytoplasm; therefore, individual cells are easily distinguishable. Preferably, the nucleus of the reporter gene-introduced cell is labeled with a spectral property different from the spectral property of the introduced reporter protein and the reporter gene-introduced cell is identified in a fluorescent or luminous nuclei image through image processing to assess the expression level of the reporter protein.

In a preferred embodiment, from the viewpoint that the reporter gene-introduced cell is easily identified in image processing by labeling the nuclei apart from light from the reporter gene in order to identify the reporter gene-introduced cell by image processing, the nuclei image identified through image processing is binary-processed, and the fluoresce or luminescence level in the area corresponding to the obtained binary image is measured for each cell to assess the expression level of the reporter gene. Generally, the spectrum of fluorescence or luminescence characteristically trails towards longer wavelengths. Then, it is advantageous that light from the nuclei has longer wavelengths than the spectrum of the signal light to be analyzed so that light (fluorescence/luminescence) from the nuclei for cell identification is not measured as signal light. In this respect, it is preferable that the nucleus is labeled with a wavelength longer than the spectral property of the reporter protein. DRAQ5 is suitable for this method for analysis because it does not require the cell to be immobilized for labeling and has a long fluorescent wavelength of approximately 680 nm. In this respect, the nucleus is preferably labeled with DRAQ5.

Preferably, from the viewpoint of an automated analytical processing, a given amount (including zero) of the chemical substance (A) or/and the inhibitor to be analyzed is previously quantified and attached inside of the wells of a microplate at the solid state and the chemical substance (A) or/and the inhibitor is melted with a given solvent for the analysis.

In the cell analysis method of the present invention, it is preferable that the expression level of the reporter gene is measured within 24 to 96 hours after the beginning of the co-culture.

### EXAMPLES

The present invention is more specifically described hereafter using examples. However, the present invention is not restricted to the examples below.

### Example 1

### Preparation of an estrogen responsive element (ERE)-containing estrogen signal reporter gene ERE-GFP and establishment of an MCF7 cell line in which the ERE-GFP is stably introduced

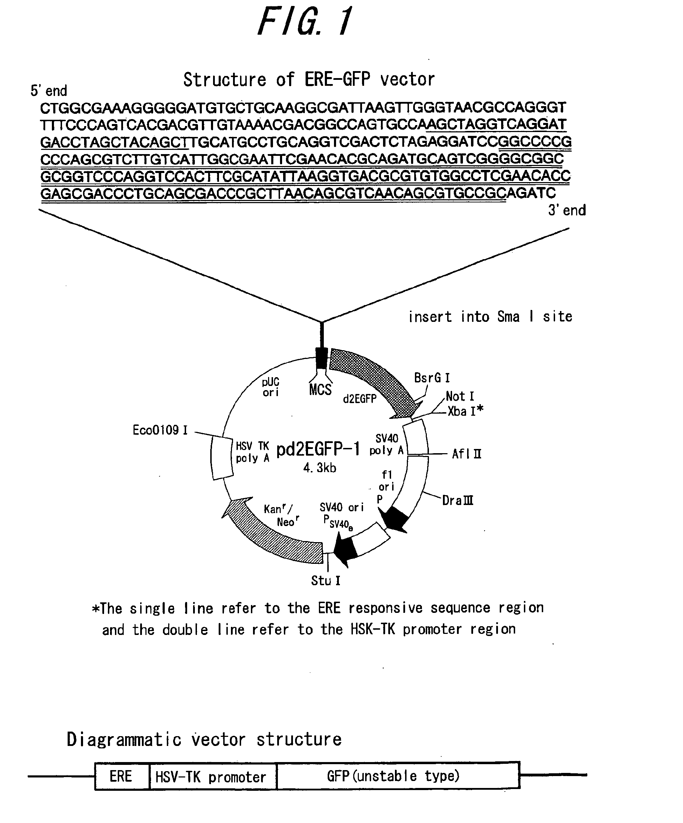

An estrogen signal reporter gene ERE-GFP was prepared as follows. A 314 bp DNA segment (SEQ. ID. No.4) containing an estrogen responsive element (AGCTAGGTCAGGATGACCTAGCTACAGCT) (SEQ. ID. No.2) and a HSV-TK promoter sequence
(GGCCCCGCCCAGCGTCTTGTCATTGGCGAATTCGAACACGCAGATGC AGTCGGGGCGGCGCGGTCCCAGGTCCACTTCGCATATTAAGGTGACGC GTGTGGCCTCGAACACCGAGCGACCCTGCAGCGACCCGCTTAACAGC GTCAACAGCGTGCCGC) (SEQ. ID. No.3) as shown in Fig.1 was introduced into a pd2EGFP-1 vector (Clontech) containing a GEP-1 gene coding an unstable green fluorescent protein (GFP) from jellyfish Aequorea having a half-life period of approximately 2 hours at the SmaI site within the multicloning site (MCS) using a standard technique to prepare a ERE-GFP gene.

The ERE-GFP gene was introduced (transfected) in a human breast cancer-derived cultured cell strain MCF7. The MCF7 cell line in the genome of which the gene is stably introduced was isolated and established. The MCF7 cell strain, which is well known for a high level of expression of estrogen receptor α (ER1 hereafter), was cultured in an RPMI-1640 culture medium containing 10% FCS in an incubator at 37 °C and with 5% CO₂.

The ERE-GFP gene was introduced into the MCF7 cell strain using the Trans IT LT-1 kit (Cat#: V2304T/Takara Shuzo). The gene was introduced according to the kit protocol. A total of 5 µl of Trans IT-LT-1 was added to 300 µl of a serum-free medium and mixed together at room temperature for 5 min and, with the addition of 1 µl of 1 to 2 µg/ml DNA, further mixed together at room temperature for 5 min. A total of 300 µl of the solution was added to 50 % confluent MCF-7 cell in a 6-cm Petri dish. Twenty four hours after the gene introduction, Geneticin was added to the culture solution to a final concentration of 1 mg/ml. From then on, the medium was replaced with the one with Geneticin every 3 to 4 days. The colony formed from a single cell was separately retrieved using a cloning ring and cultured. A total of 47 MCF7 cell lines in which the ERE-GFP gene was stably introduced were established. A MCF7 cell line in which only a pd2EGFP-1 vector having no ERE was stably introduced was similarly established as control.
Furthermore, a reporter vector in which a stable GFP having a long half-live period was used as a reporter gene and the ERE of the transcriptional control region was incorporated was also created and similarly introduced in an MCF7 cell line. Consequently, a cell line in which this reporter vector was stably introduced was established.

### Example 2

### Assessment of the estrogen signal response of the established 47 MCF7 cell lines in which the ERE-GFP gene was stably introduced

The obtained 47 MCF7 cell lines in which the ERE-GFP gene was stably introduced (ERE-GFP-E1 to E47) were assessed for the response to estrogen signal. More specifically, the cell lines were each treated with 17 β estradiol (synthetic estrogen: E₂) (+E₂ group). Their induced GFP expression levels were compared with the GFP expression levels of those treated with only the 17 β estradiol solvent (-E₂ group). When there was a significant difference in the GFP expression level between the E₂ treated and untreated groups, the clone was assumed to be well estrogen responsive and selected.

Prior to the E₂ treatment, the cell lines were each treated with trypsin and recovered. After washed with PBS, approximately 2 x 10⁵ cells were seeded in a 60 mm Petri dish. In order to eliminate the influence of estrogen-like substances contained in the 10% FCS/RPMI 1640 medium, it was cultured in a phenol red-free RPMI 1640 medium (PRF-RPMI) with the addition of 10% charcoal dextran-treated FCS (DCC-FCS) for three to four days. After the medium was replaced, E₂ was added to the culture medium to a final concentration of 10 nM (+E₂ group) and further cultured for two days. As control, a group to which only the E₂ solvent was added was prepared (-E₂ group) and further cultured for two days. Two days after, the GFP expression levels of the +E₂ and -E₂ groups of each cell line were observed under fluorescence microscope or quantified using a flow cytometer (Epics XL, Beckman Coulter).

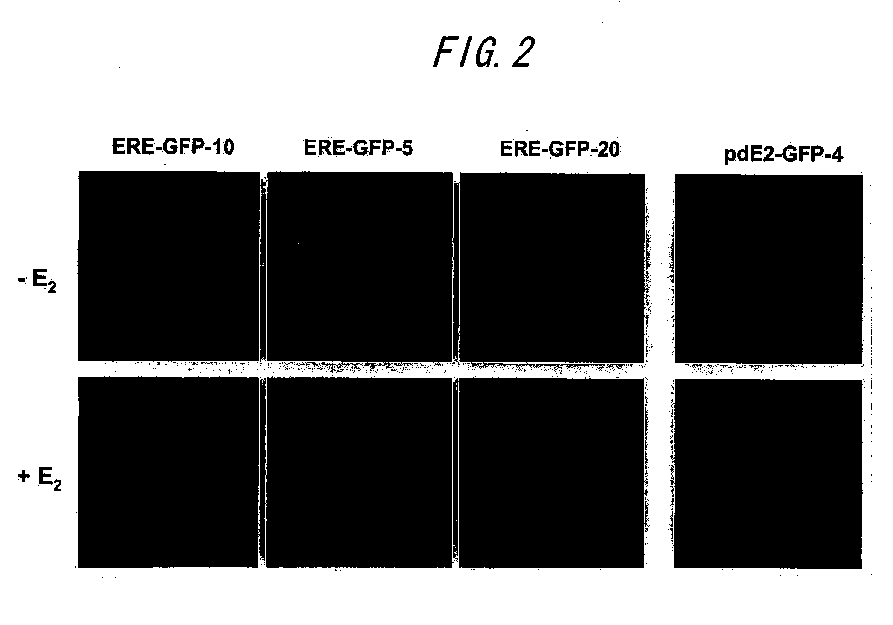

Figs. 2 and 3 show the results. The established 47 cell lines were compared with regard to the GFP expression level induced by E₂ administration. The ERE-GFP-E10 cell line (Accession No.: FERM BP-10610) had a low GFP expression level when E₂-untreated and had GFP well induced, having a high expression level when E₂-treated. Therefore, it is a preferable cell line for assessing the estrogen-induced signal response. The cell line in which only the pd2EGFP-1 vector having no ERE was stably introduced (pdE2-GFP-4) showed no GFP expression regardless of the E₂-treated/untreated. The cell line in which a reporter vector including as a reporter gene a stable GFP having a long half-life period was introduced was also analyzed with regard to the difference in GFP induction between the E₂-treated and the E₂-untreated. Table 1 shows the results of analysis.

**[Table 1]**

| Table 1: GFP protein induction rate of MCF7 cell lines (clones) in which the ERE-GFP reporter gene having a sable GFP as the reporter was stably introduced with/without the addition of estrogen (E₂) | | |
|---|---|---|
| clone No. | GFP expression positive cell rate (%) | |
| | E₂-free group | E₂-added group |
| #16 | 85 | 88 |
| #19 | 32 | 73 |
| #21 | 5 | 8 |
| #28 | 37 | 41 |
| #29 | 77 | 83 |
| #37 | 83 | 84 |

As shown in Table 1, when a stable GFP having a long half-life period was used as the reporter, there was no difference in the GFP expression level between the E₂-treated and the E₂-untreated. These cell lines are unsuitable for assessing the estrogen induced signal response.

### Example 3

### Assessment of GFP expression of the ERE-GFP-E10 cell line that is induced by the addition of E₂ and counting of living cells

The ERE-GFP-E10 cell line that was proven to be most favorable for assessing the estrogen induced signal response in the above 2 was used to obtain the living cell counts when E₂ was added at different concentrations and the GFP expressing cell counts among them.
The ERE-GFP-E10 cell was cultured in a phenol red-free RPMI 1640 medium with the addition of charcoal dextran-treated FCS to 10% (10%DCC-FCS/PRF-RPMI)) for three days and treated with trypsin for dispersion to a cell density of 1 x 10⁴ cells/mL. One mL of this cell dispersion was seeded in a 24-well multidish. E₂ was added to the culture solution to final concentrations of 0, 1, 3, 10, 30, 100, and 300 pM. After the addition, the cells were further cultured for four days. The groups to which E₂ was added to different concentrations were subjected to MTT assay for living cell counts and to fluorescence microscopy for counting GFP expressing cells.
In the MTT assay, four days after the addition of E₂, 100 µl of 5 mg/ml MTT solution was added to each well and incubated for three hours. Then, the cell solution was recovered and centrifuged at 400 Xg for 10 min. The obtained precipitate was dissolved in DMSO. The light absorbance at 560 nm of the solution was measured to obtain the living cell count.
In the GFP expressing cell counting, four days after the addition of E₂ in the same manner as in the MTT assay, the cell was treated with trypsin for dispersion. The dispersion was dropped on a slide glass and visually observed under fluorescence microscope. The cells expressing more than a specific level of GFP (GFP expression positive cells) were counted. The GFP expression positive cell count per 100 cells was obtained.

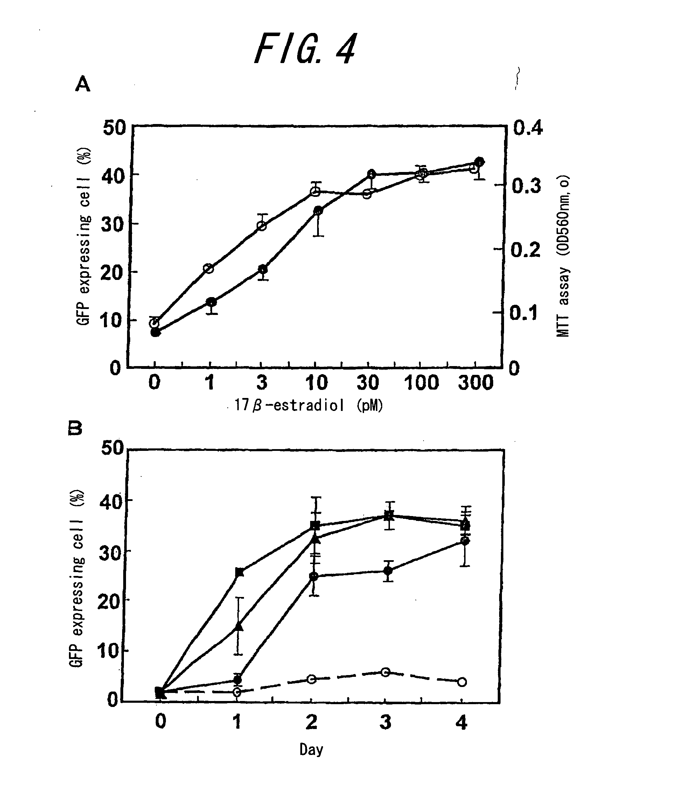

Fig.4 shows the results. In the ERE-GFP-E10 cell line, the number of cells exhibiting the E₂-induced GFP expression was increased dependently on the added E₂ concentration. In other words, a significant level of GFP expression induction was detected in the 3 pM treatment and it was maximized in the 30 pM treatment. The living cell count was also increased dependently on the added E₂ concentration. This agrees with the earlier finding in the MCF7 cell strain that the cell growth activity is increased with the addition of E₂. Fig.4B shows the results obtained by adding E₂ to a final concentration of 3 pM or 1 nM, treating the cells with trypsin for dispersion on Day 1 to Day 4, and obtaining the GFP expression positive cell count per 100 cells in the same manner as the above. Consequently, regardless of the amount of E₂ added, the GFP expression was observed from Day 1 after the addition of E₂ and the GFP expression positive cell count was maximized on Day 2.
Therefore, it was suggested that in the ERE-GFP-E10 cells established, the GFP expression is concentration-dependently induced in the added estrogen concentration range from 3 pM to 30 pM, which allows for quantitative assessment of the estrogen induced signal response.

### Example 4

### Acquisition of breast cancer tissue and isolation and culture of adipose stromal cells from the tissue

A surgically removed breast cancer tissue specimen was divided. A part was used for histopathological diagnosis and another part was used for isolation and culture of the adipose stromal cell. The adipose stromal cell was isolated from the specimen and cultured as follows. A piece of tissue was immersed in HBSS, rinsed, and chopped by postmortem scissors to a size smaller than 1 mm³, incubated in a HBSS solution with the addition of 1 mg/ml of collagenase, 40 mg/ml of BSA, 2 mg/ml of glucose, 1X antibiotic-antimycotic, and 50 µg/mL of gentamicin at 37 °C for two to three hours to further disperse the cells. The cell dispersion was filtered with a nylon mesh. The filtrate containing the dispersed cell was precipitated by centrifugal and rinsed with HBSS several times to recover the breast cancer tissue-derived cell. The cell was suspended in a 10% FCS added MEM-α medium and cultured in an incubator at 37 °C and with 5% CO₂. The following day, the medium was replaced to remove non-adhesive cells. Seven to ten days after the culture started, the growth of the adipose stromal cell was observed. From then on, the culture solution was replaced with a fresh culture solution two times a week.

### Example 5

### Detection of estrogen signal induced from a breast cancer adipose stromal cell by co-culturing the breast cancer tissue-derived adipose stromal cell and MCF7 cell line in which ERE-GFP is stably introduced ERE-GFP-E10

A breast cancer tissue-derived adipose stromal cell and the MCF7 cell line in which the ERE-GFP is stably introduced ERE-GFP-E10 were co-cultured in a charcoal-dextran treated, 10% FCS added, and phenol red-free RPMI 1640 medium (10%DCC-FCS/PRF-RPMI). The collected adipose stromal cell was used within two or three passages after the culture started to minimize the influence of aging.

Prior to the co-culturing, the ERE-GFP-E10 cell was cultured in a 10% DCC-FCS/PRF-RPMI culture medium (termed the estrogen depleted medium hereafter). The adipose stromal cell was prepared in the depleted medium to a cell density of 5 x 10⁴ cells/mL. One ml of this cell dispersion was seeded in a 24-well multidish to culture. On Day 1 after the adipose stromal cell was seeded in the wells to culture, the ERE-GRE-E10 cell was added on the adipose stromal cell to 5 × 10⁴ cells per well and co-cultured for four days with the concurrent addition of testosterone, which is the substrate for estrogen production reaction by the aromatase enzyme, to a final concentration of 10⁻⁷ pM. After the four days of co-culturing, the cell was gently treated with trypsin for dispersion. The GFP expression positive cell was detected under microscope. The ERE-GFP-E10 cell and adipose stromal cell were distinguishable under microscope by their shapes.

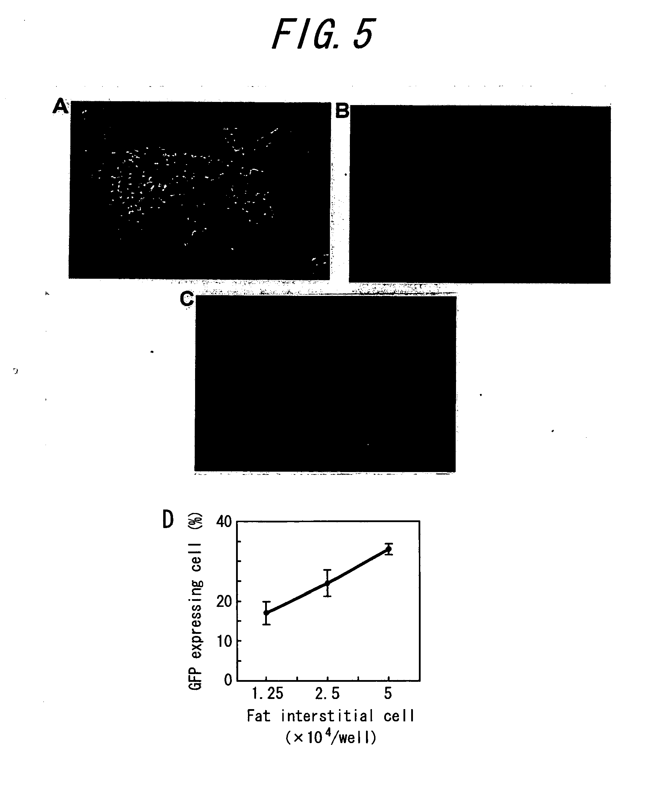

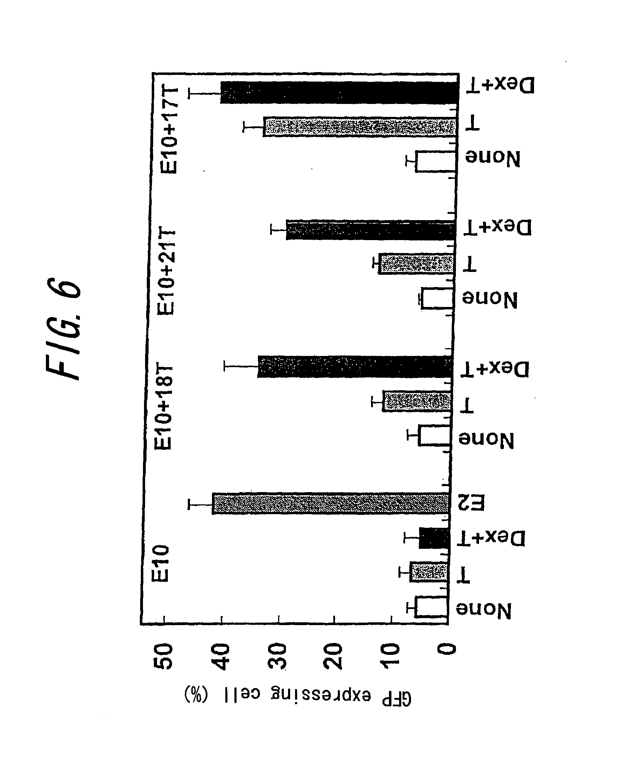

Fig.5 shows the GFP expression induction in the ERE-GFP-E10 cell after the breast cancer tissue-derived adipose stromal cell and ERE-GFP-E10 cell were co-cultured. When the breast cancer tissue-derived adipose stromal cell and ERE-GFP-E10 cell were co-cultured with the addition of testosterone, which is the substrate of estrogen production reaction by the aromatase enzyme, the GFP expression was induced in the ERE-GFP-E10 cell. Furthermore, the GFP expression positive cell count was increased dependently on the number of co-cultured breast cancer tissue-derived adipose stromal cells.
Furthermore, the GFP positive cell rate in the ERE-GFP-E10 cell was calculated by using the same process for the adipose stromal cells collected from three breast cancer cases with the addition or no addition of testosterone, or with the addition of testosterone plus 2 x 10⁻⁶ M dexamethasone, which is an aromatase inducer. Fig.6 shows the results. The GFP positive cell rate in the ERE-GFPE 10 cell was significantly increased after co-cultured with the adipose stromal cell with the addition of androgen compared with the case of no addition of androgen. In the groups where dexamethasone or an aromatase inducer was further added, the GFP positive cell rate was further increased compared with the groups of androgen by itself.
From the above, when the breast cancer tissue-derived adipose stromal cell and ERE-GFP-E10 cell are co-cultured in the presence of testosterone, which is the substrate of estrogen production reaction by the aromatase enzyme, the GFP expression positive cell rate in the ERE-GFP-E10 cell is increased dependently on the number of co-cultured adipose stromal cells. Therefore, the estrogen signal induced by the adipose stromal cell via estrogen production reaction by the aromatase enzyme can quantitatively be measured. Furthermore, it was suggested that the induced estrogen signal varies among cancer cases. It was also suggested that the estrogen signal is mainly caused by the aromatase activity in the adipose stromal cell since it is enhanced by the addition of dexamethasone.

### Example 6

### Analysis of estrogen signal intensity induced by the breast cancer adipose stromal cell in cancer cases by co-culturing the breast cancer tissue-derived adipose stromal cell and MCF7 cell line in which the ERE-GFP is stably introduced ERE-GFP-E10

Breast cancer tissue-derived adipose stromal cells collected from two sites of each of different breast cancer cases and the MCF7 cell line in which the ERE-GFP is stably introduced ERE-GFP-E10 were co-cultured to quantify the estrogen signal induced by the adipose stromal cell in the individual cancer cases. The co-culture was conducted in a charcoal dextran treated, 10% FCS added, and phenol red-free RPMI 1640 medium (10%DCC-FCS/PRM-RPMI). The collected adipose stromal cells were used within two or three passages after the culture started to minimize the influence of aging.
In the same manner as the above example, prior to the co-culturing, the ERE-GFP-E10 cell was cultured in a 10%DCC-FCS/PRF-RPMI culture medium (termed the estrogen depleted medium hereafter). The adipose stromal cell was prepared in the depleted medium to a cell density of 5 x 10⁴ cells/mL. One ml of this cell dispersion was seeded in a 24-well multidish to culture. On Day 1 after the adipose stromal cell was seeded in the wells to culture, the ERE-GRE-E10 cell was added on the adipose stromal cell to 5 x 10⁴ cells per well and co-cultured for four days with the concurrent addition of testosterone, which is the substrate for estrogen production reaction by the aromatase enzyme, to a final concentration of 10⁻⁷ pM. After the four days of co-culturing, the cell was gently treated with trypsin for dispersion. The GFP expression positive cell was detected under microscope. The ERE-GFP-E10 cell and adipose stromal cell were distinguishable under microscope by their shapes.

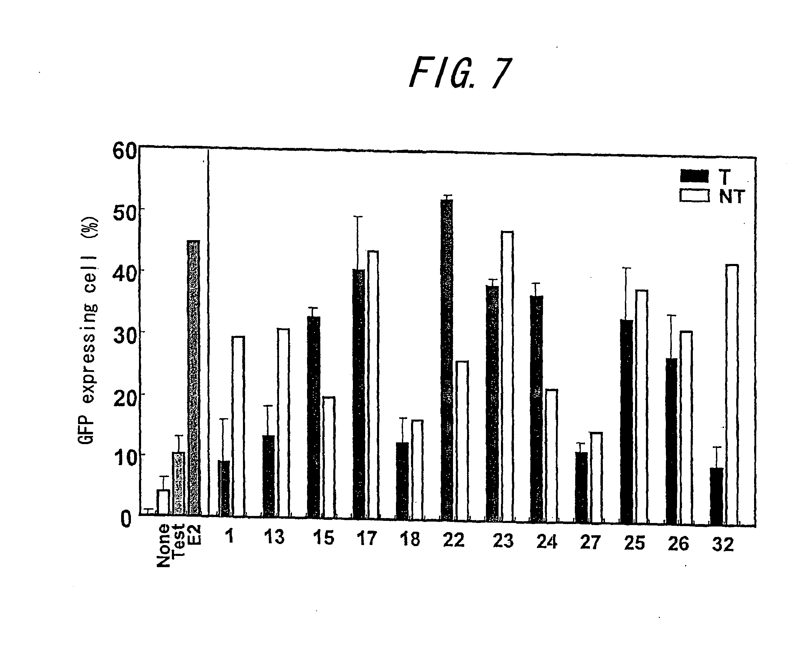

Fig.7 shows the GFP expression induction in the ERE-GFP-E10 cell after the breast cancer tissue-derived adipose stromal cell collected from two sites of each of cancer cases and the ERE-GFP-E10 cell were co-cultured. The adipose stromal cells were collected from the removed cancer tissue of each case at two sites, the cancer site and a site 2 cm away from the cancer site. Consequently, the estrogen signal induced by the adipose stromal cell varied among the cancer cases. The estrogen signal varied even in the same case depending on the collected sites. This suggests that the estrogen signal is induced by the adipose stromal cell at different intensities in individual breast cancer cases and, therefore, the sensitivity to hormonal therapy using an aromatase inhibitor may vary.

### Example 7

### Comparison between estrogen signal intensity induced by the breast cancer adipose stromal cell with and aromatase gene mRNA expression level in the stromal cell in cancer cases, quantified by co-culturing the breast cancer tissue-derived adipose stromal cell and MCF7 cell line in which the ERE-GFP is stably introduced ERE-GFP-E10

Breast cancer tissue-derived adipose stromal cells collected from different breast cancer cases and the MCF7 cell line in which the ERE-GFP is stably introduced ERE-GFP-E10 were co-cultured to quantify the estrogen signal from the adipose stromal cell in individual cancer cases. The same process as the above example was used. The expression level of aromatase gene mRNA was analyzed for the adipose stromal cell from the same source using the quantitative RT-PCR technique.

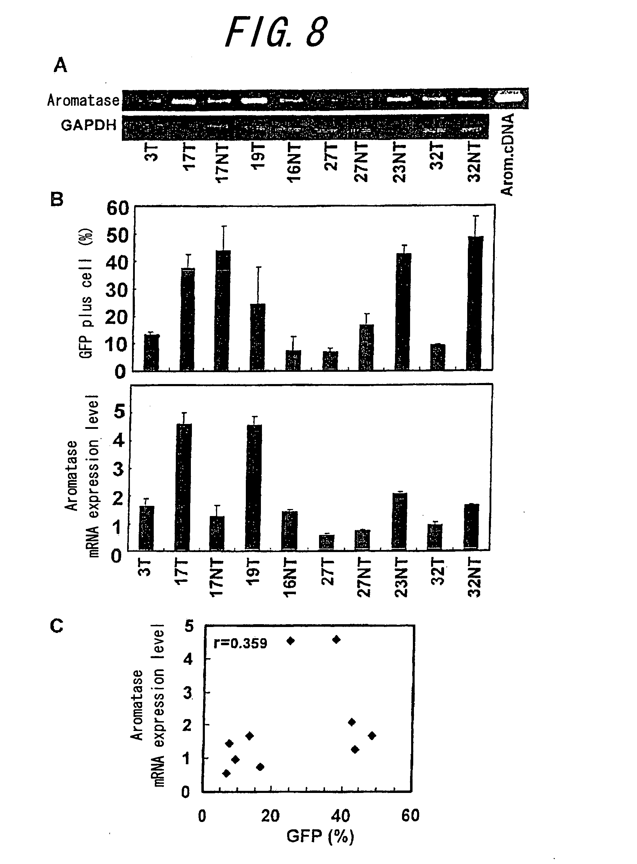

Fig.8 shows the GFP expression induction and the quantified aromatase gene mRNA in the adipose stromal cell of the ERE-GFP-E10 cell after the breast cancer tissue-derived adipose stromal cell and ERE-GFP-E10 cell were co-cultured for ten breast cancer cases. The results suggest that the estrogen signal induced by the adipose stromal cell varied among the cancer cases and the estrogen signal intensity and aromatase gene mRNA expression level were not always correlated with each other. This suggests that the substantial estrogen signal intensity induced by the adipose stromal tissue possibly cannot be assessed by simply quantifying the aromatase gene mRNA level in the adipose stromal tissue because the estrogen signal intensity induced by the adipose stromal cell varies among individual breast cancer cases and the estrogen signal intensity and aromatase gene mRNA expression level are not always correlated with each other.

### Example 8

### Analysis of estrogen signal intensity induced by co-culturing a breast cancer tissue-derived adipose stromal cell and the MCF7 cell line in which the ERE-GFP is stably introduced ERE-GFP-E10 in the presence of various aromatase inhibitors and assessment of estrogen signal inhibition effect of the aromatase inhibitors

Breast cancer tissue-derived adipose stromal cells collected from four breast cancer cases and the MCF7 cell line in which the ERE-GFP is stably introduced ERE-GFP-E10 were co-cultured under a total of four different conditions, namely with the addition of testosterone and an aromatase inhibitor at four different concentrations (0, 0.01, 0.03, and 0.1 µM). The estrogen signal from the adipose stromal cell under each condition was quantified. Three aromatase inhibitors, anastrozol, retrozol, and exemesthane, were used. The experiment was conducted for each inhibitor.

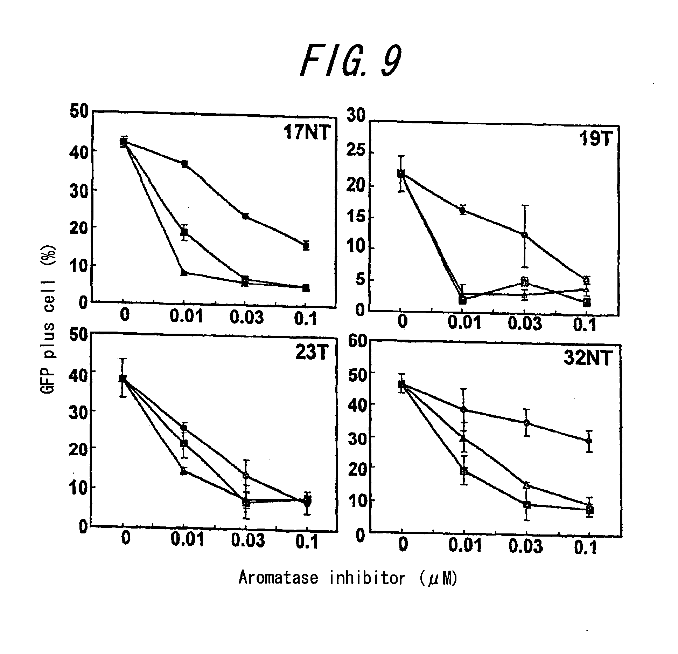

Fig.9 shows the results. The rate of GFP positive cell was decreased dependently on the added aromatase inhibitor concentration in the adipose stromal cells of all cases. The rate of GFP positive cell was decreased differently among the breast cancer cases and even in the same breast cancer case depending on the added aromatase inhibitors. Therefore, it is suggested that the estrogen signal induced by the adipose stromal cell varies among breast cancer cases and the suppression effect of various aromatase inhibitors on the estrogen signal varies among cases and that, using this technique, the suppression effect of various aromatase inhibitors on estrogen signal in individual cancer cases can quantitatively be assessed and the sensitivity to hormonal therapy using various aromatase inhibitors can possibly be assessed in individual cases.

### Example 9

### Quantitative detection of estrogen signal induction by breast cancer tissue using supernatant extracted from surgically removed breast cancer tissue specimen

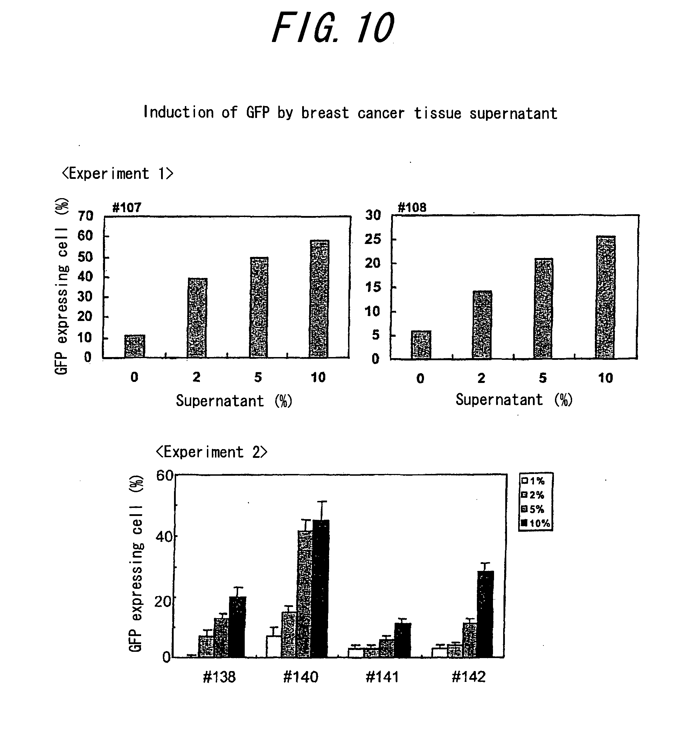

A breast cancer tissue (50mg/ml) was sliced by scissors to a size of 1 to 2 mm³ in a phenol red-free RPMI 1640 medium and centrifuged (12,000 rpm for 10 min) to prepare a supernatant. The supernatant was added to the ERE-GFP-E10 cell (3 x 10⁴ cells/ml, 24-well culture dish) at a concentration of 20 to 100 % in a depleted RPMI 1640 medium containing a dextran-charcoal treated serum (10%), and cultured for four days. Then, GFP expressing E10 cells were counted under fluorescence microscope to determine the positive rate. Fig. 10 shows the results. It is understood from Fig. 10 that the number of GFP expression positive cells was increased as the supernatant concentration was raised, namely dose-dependently. Furthermore, it was suggested that the estrogen signal intensity varied among cases (#107, #108, #138, #140, #141, and #142 in Fig.10).

### Example 10

### Quantitative detection of estrogen signal induction by breast cancer tissue by co-culturing with surgically removed and sliced breast cancer tissue specimen using an insert well

### GFP expression induction by breast cancer tissue

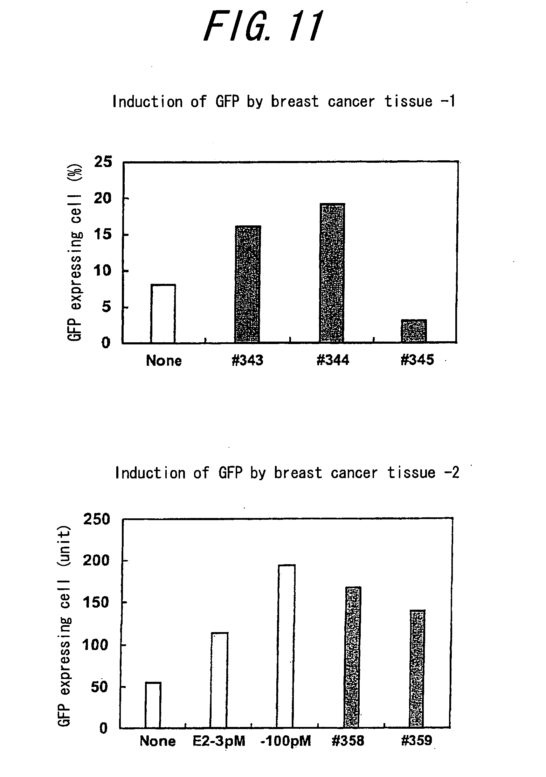

A breast cancer tissue (50mg/ml) was sliced by scissors to a size of 1 to 2 mm³ in a phenol red-free RPMI 1640 medium. The obtained suspension was added to the ERE-GFP-E10 cell (3 × 10⁴ cells/ml, 24-well culture dish) in a depleted RPMI 1640 medium containing a dextran-charcoal treated serum (10%) and cultured. Four days later, GFP expressing E10 cells were counted under fluorescence microscope to determine the positive rate. Fig. 11 shows the results. Fig. 11 suggests that that the estrogen signal intensity varied among cases (#343, #344, #345, #358, abd #359 n Fig.11).

### Example 11

Then, a method for quantitatively analyzing the aromatase production ability of stromal cells isolated from breast cancer tissue and the effectiveness of aromatase inhibitors using a fluorescent protein gene-introduced breast cancer cell prepared by introducing in a breast cancer cell a fluorescent protein-coding gene as a reporter protein-coding gene was examined.

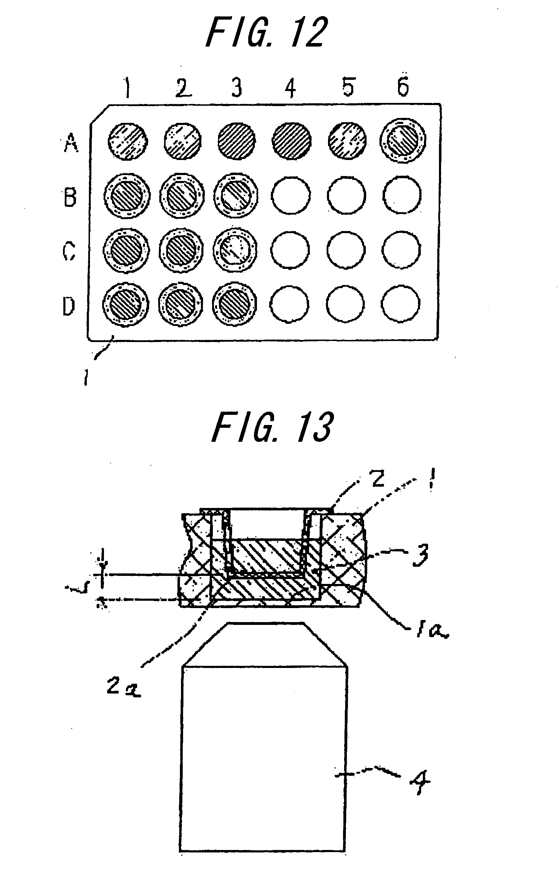

In Fig. 12, a microplate 1 has 24 wells (A1 to D6). Interstitial cell samples isolated from breast cancer tissues surgically removed from patients were grouped as follows.
A1 to A6: these wells were examined under the conditions below for assessing the estrogen production ability of the adipose stromal cells. The wells contained the same culture medium containing androgen.
A1: only breast cancer cell introduced with fluorescent protein gene
A2: breast cancer cell introduced with fluorescent protein gene + estrogen (0.3 pM)
A3: breast cancer cell introduced with fluorescent protein gene + estrogen (1 pM)
A4: breast cancer cell introduced with fluorescent protein gene + estrogen (3 pM)
A5: breast cancer cell introduced with fluorescent protein gene + estrogen (10 pM)
A6: breast cancer cell introduced with fluorescent protein gene + stromal cell
B 1 to 3, C 1 to 3, D 1 to 3: these wells were examined under the conditions bellow for quantitatively analyzing the effectiveness of three aromatase inhibitors X, Y, and Z.
B1: breast cancer cell introduced with fluorescent protein gene + stromal cell + aromatase inhibitor X (10 pM)
B2: breast cancer cell introduced with fluorescent protein gene + stromal cell + aromatase inhibitor X (30 pM)
B3: breast cancer cell introduced with fluorescent protein gene + stromal cell + aromatase inhibitor X (100 pM)
C1: breast cancer cell introduced with fluorescent protein gene + stromal cell + aromatase inhibitor Y (10 pM)
C2: breast cancer cell introduced with fluorescent protein gene + stromal cell + aromatase inhibitor Y (30 pM)
C3: breast cancer cell introduced with fluorescent protein gene + stromal cell + aromatase inhibitor Y (100 pM)
D1: breast cancer cell introduced with fluorescent protein gene + stromal cell + aromatase inhibitor Z (10 pM)
D2: breast cancer cell introduced with fluorescent protein gene + stromal cell + aromatase inhibitor Z (30 pM)
D3: breast cancer cell introduced with fluorescent protein gene + stromal cell + aromatase inhibitor Z (100 pM)
The breast cancer cell introduced with fluorescent protein gene and stromal cell were cultured in the wells of the microplate as follows.

### <Structure>

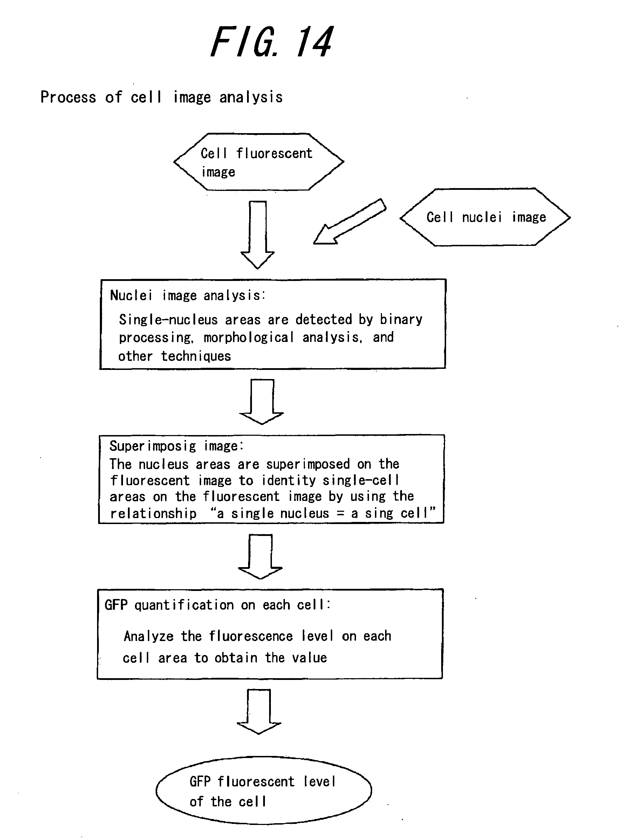

Fig. 13 is a partial cross-sectional view of the microplate. As shown in this figure, the breast cancer cell introduced with fluorescent protein gene is cultured at the bottom 1a of the microplate 1. The stromal cell is co-cultured in a culture solution 3 containing at least androgen on the base 2a of an insert 2 consisting of a membrane having a culture surface. A fluorescent image of the breast cancer cell introduced with fluorescent protein gene is captured via an objective lens 4 of a not-shown inverted microscope. The objective lens 4 is focused on the bottom 1a of the microplate where the breast cancer cell introduced with fluorescent protein gene is cultured. The objective lens 4 is driven by a not-shown electrically operated stage in relation to the wells. A camera (not-shown) mounted on the microscope generally captures approximately 4 to 16 fluorescent images to measure the average fluorescent brightness of breast cancer cell introduced with fluorescent protein gene.
The insert 2 is detachably provided. It is detached before the images are captured. In this way, stray light from the insert base 2a is eliminated and the fluorescent images have a low background. Furthermore, the distance between the insert base 2a and the bottom 1a of the microplate 1 was set to be not within the range of the focal length of the objective lens 4; then, fluorescent images of breast cancer cell introduced with fluorescent protein gene can be obtained without detaching the insert 2, easily automating the above cell analysis process (see Fig.14).
The present invention is not restricted to the above embodiment and various embodiments are available. In this embodiment, a microscope is used to capture a fluorescent image and the fluorescence level is assessed based on the obtained image. A flow cytometer can be used to assess the fluorescence level.

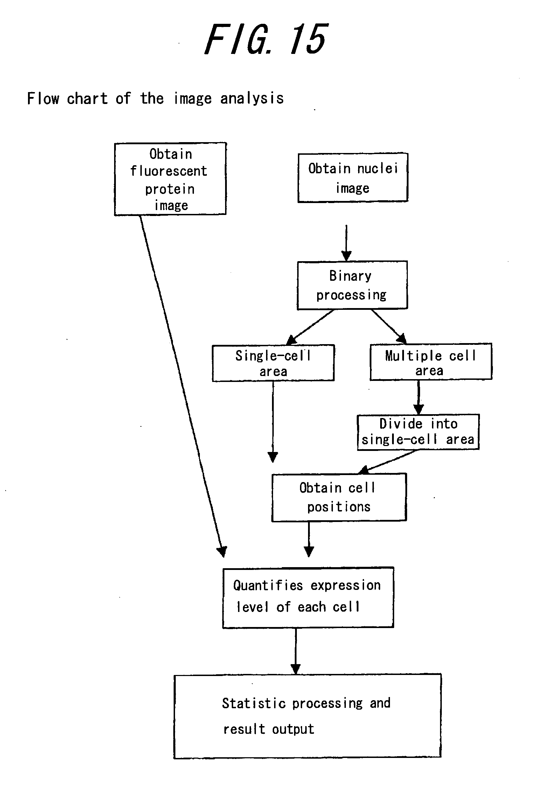

An embodiment of the method for quantitatively assessing the expression level of the reporter protein according to Claims 41 to 43 in which a fluorescent protein is used as the reporter protein is described along with (Fig.15: image analysis flow).

The expression level of introduced fluorescent protein is quantified by analyzing a cell image obtained by the above apparatus to identify the position or boundary of each cell in the image and calculating the brightness of the image in the area at that position or enclosed by the boundary.

The cell is identified mainly using a nuclei image obtained separately from the fluorescent protein image.

The cell is identified by converting the brightness of the nuclei image into binary data using a proper brightness as the threshold and specifying a target nucleus area.

Only partial areas having a certain size are selected in the binary image. The size is determined for example according to the largest or smallest width of the area or the diameter of the largest inscribing circle within the area.

The above selection results in eliminating non-cell areas such as dust in the cell nuclei image.

When the cell to be analyzed is present at a low density, the cells are observed separately in the cell image. Therefore, the areas obtained in the above process each presumably correspond to a single nucleus. They are assumed to be a single cell and the nucleus center position is obtained.

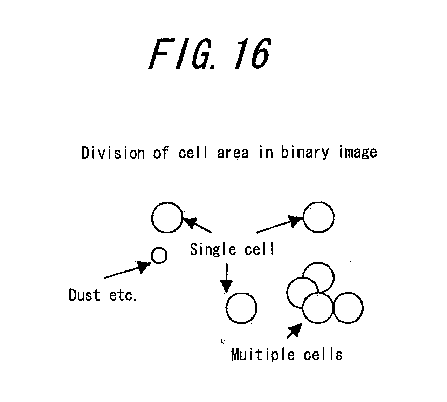

When the cell to be analyzed is present at a high density, multiple cells are in contact with or overlapped with each other. In this case, the degree of roundness, or an indicator of how circular the area is, is defined and the areas having high degrees of roundness are assumed to be a single cell area and the areas having low degrees of roundness are assumed to be a multiple-cell area (Fig. 16: division of cell areas in a binary image).

For the multiple-cell area, the individual cells are separated in the process below to identify the position and size of a single cell.

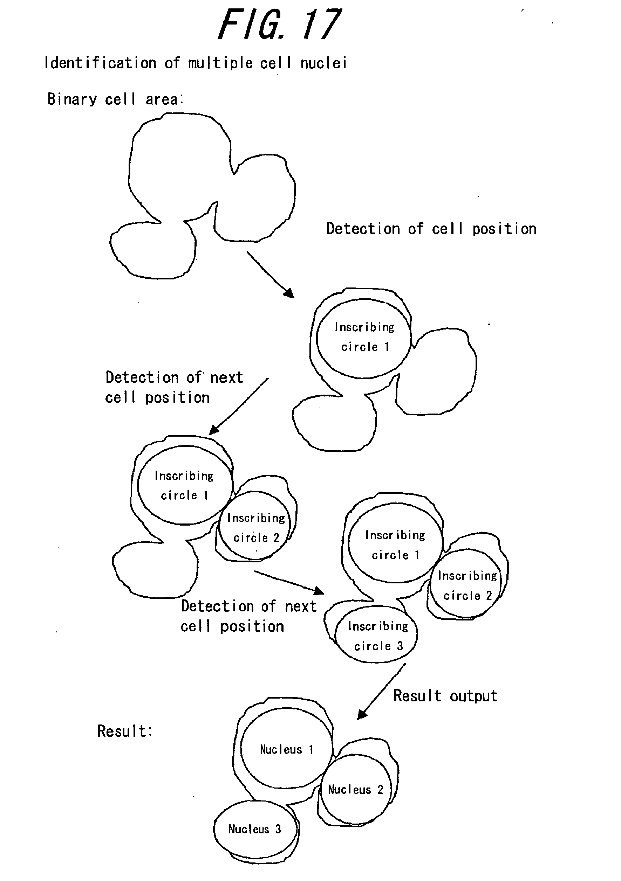

Multiple cells are separated for example as follows (Fig. 17: recognition of multiple cell nuclei).

It is determined whether or not an inscribing circle approximately the size of a standard cell nucleus fits in a bounded binary area presumably containing multiple cell nuclei. The larger inscribing circle is first used to fill the area. This is repeated until no inscribing circle of the minimum nucleus size or larger can fill the area. In this way, individual cell positions are determined.

The fluorescence expression level of each cell is quantified with cross-reference to the cell nucleus positions, sizes, and forms obtained in the above process and the fluorescent protein image.

The obtained fluorescence expression level is used to quantify the effectiveness of inhibitors after statically processed such as averaging and cutting off of lower florescent levels.

### <Analysis>

It was found that the estrogen production ability can quantitatively be analyzed by comparing the average fluorescence brightness of the breast cancer cell introduced with fluorescent protein gene in the well A6 (the breast cancer cell introduced with fluorescent protein gene + stromal cell) with the average fluorescence brightness of the wells A1 to A5. Furthermore, it was found that the effectiveness of aromatase inhibitors can quantitatively be analyzed by comparing the average fluorescence brightness of the breast cancer cell introduced with fluorescent protein gene in the well A6 (the breast cancer cell introduced with fluorescent protein gene + stromal cell) with the average fluorescence brightness of the wells B 1 to 3, C 1 to 3, D 1 to 3 and with the average fluorescence brightness of the wells A1 to A5.

BUDAPEST TREATY ON THE INTERNATIONAL
RECOGNITION OF THE DEPOSIT OF MICROORGANISMS
FOR THE PURPOSE OF PATENT PROCEDURE

RECEIPT IN THE CASE OF AN ORIGINAL DEPOSIT issued pursuant to Rule 7.1
by the INTERNATIONAL AUTHORITY identified at the bottom of this page

Receipt of deposition on the original deposition

NAME OF DEPOSITOR

Saitama Prefectural Cancer Center
Institute of Clinical Cancer Research
Specialized Researcher, Ms. Yuri Yamaguchi

ADDRESS OF DEPOSITOR

Komuro 818, Ina-cyo, Kitaadachi-gun, Saitama-ken, 362-0806, Japan

I. IDENTIFICATION OF THE MICROORGAMISM
Identification reference given by the | Accession number given by the
DEPOSITOR: | International Depository Authority :
MCF-7-E10 | FERM BP-10610
II. SCIENTIFIC DESCRIPTION AND/OR PROPOSED TAXONOMIC DESIGNATIOM
The microorganism identified under I above was accompanied by :
☒ A scientific description
☒ A proposed taxonomic designation
(Mark with a cross where applicable)
III. RECEIPT AND ACCEPTANCE
The International Depository authority accepts the microorganism identified under I
above, which was received by it on
IV. RECEIPT OF REQUEST FOR CONVERSION
The microorganism identified I above was received by this International Depository
Authority on May 25, 2005 (date of the original deposit).
(A request to convert to a deposit under the Budapest Treaty was received from the
original deposition of FERM P-20546 deposited at May 25, 2005)
VI. INTERNATIONAL DEPOSITORY AUTHORITY
Name: International Patent Depository Authority
National Institute of Advanced Industrial Science and Technology
Dr. Masakazu Yamaoka, Director
Address: AIST Tsukuba Central 6, 1-1, Higashi 1-chome Tsukuba-shi,
Ibaraki-ken 305-8566 Japan
Date May 22, 2006

BUDAPEST TREATY ON THE INTERNATIONAL
RECOGNITION OF THE DEPOSIT OF MICROORGANISMS
FOR THE PURPOSE OF PATENT PROCEDURE

VIABILITY STATEMENT
Issued pursuant Rule 10.2 by the INTERNATIONAL
DEPOSITORY AYTHORITY identified on the following page

Certification of the viability of the organism

NAME OF DEPOSITOR

Saitama Prefectural Cancer Center
Institute of Clinical Cancer Research
Specialized Researcher, Ms. Yuri Yamaguchi

ADDRESS OF DEPOSITOR

Komuro 818, Ina-cyo, Kitaadachi-gun, Saitama-ken, 362-0806, Japan

1. DEPOSITOR | 2. IDENTIFICATION OF THE MICROORGAMISM
Name:
Saitama Prefectural Cancer Center
Institute of Clinical Cancer Research
Specialized Researcher,
Ms. Yuri Yamaguchi
Address:
Komuro 818, Ina-cyo, Kitaadachi-gun,
Saitama-ken, 362-0806, Japan | Accession number given by the
International Depository Authority :
FERM BP-10610
Date of the deposit or the transfer:
May 25, 2005
3. VIABILITY STATEMENT
The viability of the microorganism identified under 2 above was tested May 9, 2006.
On that date, the said microorganism was
☒ viable
☐ no longer viable

4. CONDITION UNDER WHICH THE VIABILITY TEST WAS PERFORMED (Only
when the result was negative)
☐ Record of the condition of the microorganism One copy
5. INTERNATIONAL DEPOSITORY AUTHORITY
Name: International Patent Depository Authority
National Institute of Advanced Industrial Science and Technology
Dr. Masakazu Yamaoka, Director
Address: AIST Tsukuba Central 6, 1-1, Higashi 1-chome Tsukuba-shi,
Ibaraki-ken 305-8566 Japan
Date July 20, 2006

## Claims

1. A transgenic cell **characterized in that** a reporter vector containing a gene coding a reporter protein and an estrogen responsive element (ERE) at the upstream of the promoter region of the reporter protein, wherein the expression of the reporter protein is substantially controlled by the estrogen responsive element is stably introduced.

2. The cell according to Claim 1 or 2 wherein the cell in which the reporter vector is stably introduced is a human breast cancer-derived cultured cell strain.

3. The cell according to any one of Claims 1 to 3 wherein the cell in which the reporter vector is stably introduced is a human breast cancer-derived cultured cell strain MCF7, in which a high level of estrogen receptor is intrinsically expressed.

4. The cell according to any one of Claims 1 to 3 **characterized in that** the reporter protein is a protein derived from at least one selected from the group consisting of fluorescent proteins such as GFP and RFP, or from the group consisting of enzymes such as luciferase, β-galactosidase, β-lactamase, and β-glucuronidase.

5. The cell according to any one of Claims 1 to 3 **characterized in that** the reporter protein is a protein derived from at least one selected from the group consisting of fluorescent proteins such as GFP and RFP, or from the group consisting of enzymes such as luciferase, β-galactosidase, β-lactamase, and β-glucuronidase, and the half-life period of the protein is approximately 2 hours to 6 hours.

6. The cell according to any one of Claims 1 to 5 wherein said cell exhibits a difference in the level of reporter gene expression to the extent that it is visualized or distinguished between the estrogen treated case and the estrogen untreated case.

7. The cell according to Claim 6 wherein said difference is the difference between the reporter gene expression intensity for the maximum cell number in the estrogen untreated case and the reporter gene expression intensity for the maximum cell number in the estrogen treated case.

8. The cell according to any one of Claims 1 to 7 wherein with the borderline being set by said reporter gene expression intensity for the maximum cell number in the estrogen treated case, a sufficient number of cells having a high level of expression intensity are present to the extent to achieve visualization by said reporter gene.

9. The cell according to any one of Claims 1 to 7 **characterized in that** when said cell is treated by estrogen, the reporter gene expression level of said cell is changed dependently on the estrogen concentration treated.

10. The cell according to any one of Claims 1 to 9 wherein the transgenic cell is the cell deposited under accession number FERM BP-10610.

11. A method for assessing estrogen signal wherein the assessment is achieved by visualizing the estrogen signal response using the cell according to any one of Claims 1 to 10.

12. A method for quantitatively assessing the induction of estrogen signal mainly via aromatase enzymatic activity derived from a breast cancer tissue-derived adipose stromal cell, the method comprising the following steps of:
A: co-culturing the cell according to any one of Claims 1 to 10 and a breast cancer tissue -derived adipose stromal cell;
B: adding androgen, which is the substrate for aromatase, to said two co-cultured cells and culturing them;
C: quantifying the reporter protein induced by estrogen that is produced by the adipose stromal cell aromatase and released into the culture medium; and
D: quantitatively assessing the induction of estrogen signal mainly via aromatase enzymatic activity of the adipose stromal cell based on the amount of the reporter protein quantified in said step C.

13. The method for assessing according to Claim 12 **characterized in that** said step A comprises culturing the cell according to any one of Claims 1 to 10 at the bottom of a culture vessel, adding a breast cancer tissue section obtained by slicing the breast cancer tissue containing adipose stromal cell to the culture vessel using an insert and co-culturing the cell according to any one of Claims 1 to 10 and the breast cancer tissue-derived adipose stromal cell.

14. A method for quantitatively assessing the induction of estrogen signal mainly via aromatase enzymatic activity derived from a breast cancer tissue-derived adipose stromal cell, the method comprising the following steps of:
A: slicing a breast cancer tissue containing adipose stromal cell in a culture medium and then centrifuging the medium to obtain a supernatant;
B: adding said supernatant and androgen, which is the substrate for aromatase, to the cell according to any one of Claims 1 to 10;
C: quantifying the reporter protein induced by estrogen produced by the adipose stromal cell aromatase and released into the culture solution; and
D: quantitatively assessing the induction of estrogen signal mainly via aromatase enzymatic activity of the adipose stromal cell based on the amount of the reporter protein quantified in said step C.

15. A method for quantitatively assessing the induction of estrogen signal mainly via aromatase enzymatic activity that is induced from a breast cancer tissue-derived adipose stromal cell, the method comprising the following steps of:
A: co-culturing the cell according to any one of Claims 1 to 10 and a breast cancer tissue-derived adipose stromal cell;
B: adding androgen (testosterone), which is the substrate for aromatase, to said two co-cultured cells;
C: quantifying the reporter protein induced by both or either one of the estrogen signal induced by estrogen produced by the adipose stromal cell aromatase and released into the culture medium and an estrogen receptor activation mechanism induced from the adipose stromal cell and having no relation with aromatase enzyme; and
D: quantitatively assessing the induction of estrogen signal mainly via aromatase enzymatic activity of the adipose stromal cell based on the amount of the reporter protein quantified in said step C.

16. The method for assessing according to Claim 15 **characterized in that** said step A comprises culturing the cell according to any one of Claims 1 to 10 at the bottom of a culture vessel, adding a breast cancer tissue section obtained by slicing the breast cancer tissue containing adipose stromal cell to the culture vessel using an insert and co-culturing the cell according to any one of Claims 1 to 10 and the breast cancer tissue-derived adipose stromal cell.

17. A method for quantitatively assessing the induction of estrogen signal mainly via aromatase enzymatic activity that is derived from a breast cancer tissue-derived adipose stromal cell, the method comprising the following steps of:
A: slicing a breast cancer tissue containing adipose stromal cell in a culture medium and then centrifuging the medium to obtain a supernatant;
B: adding said supernatant and androgen, which is the substrate for aromatase, to the cell according to any one of Claims 1 to 10;
C: quantifying the reporter protein induced by both or either one of the estrogen signal induced by estrogen produced by the adipose stromal cell aromatase and released into the culture medium and an estrogen receptor activation mechanism induced from the adipose stromal cell and having no relation with aromatase enzyme; and
D: quantitatively assessing the induction of estrogen signal mainly via aromatase enzymatic activity of the adipose stromal cell based on the amount of the reporter protein quantified in said step C.

18. A method for quantitatively assessing the inhibitory effect of an aromatase inhibitor on estrogen signal mainly via aromatase enzymatic activity that is derived from a breast cancer tissue-derived adipose stromal cell, the method comprising the following steps of:
A: co-culturing the cell according to any one of Claims 1 to 10 and the breast cancer tissue-derived adipose stromal cell;
B: simultaneously adding androgen, which is the substrate for aromatase, and an aromatase inhibitor to said two co-cultured cells;
C: quantifying the reporter protein induced by estrogen produced by the adipose stromal cell aromatase and released into the culture medium; and
D: quantitatively assessing the inhibitory effect of the aromatase inhibitor on estrogen signal mainly via aromatase enzymatic activity of the adipose stromal cell based on the amount of reporter protein quantified in said step C.

19. The method for assessing according to Claim 18 **characterized in that** said step A comprises culturing the cell according to any one of Claims 1 to 10 at the bottom of a culture vessel, adding a breast cancer tissue section obtained by slicing the breast cancer tissue containing adipose stromal cell to the culture vessel using an insert and co-culturing the cell according to any one of Claims 1 to 10 and the breast cancer tissue-derived adipose stromal cell.

20. A method for quantitatively assessing the inhibitory effect of an aromatase inhibitor on estrogen signal mainly via aromatase enzymatic activity that is derived from a breast cancer tissue-derived adipose stromal cell, the method comprising the following steps of:
A: slicing a breast cancer tissue containing adipose stromal cell in a culture medium and then centrifuging the medium to obtain a supernatant;
B: adding said supernatant and androgen, which is the substrate for aromatase, to the cell according to any one of Claims 1 to 10;
C: quantifying the reporter protein induced by estrogen produced by the adipose stromal cell aromatase and released into the culture medium; and
D: quantitatively assessing the inhibitory effect of the aromatase inhibitor on estrogen signal mainly via aromatase enzymatic activity of the adipose stromal cell based on the amount of the reporter protein quantified in said step C.

21. A method for analysis of an inhibitor wherein an aromatase inhibitor is analyzed as to whether or not it has a tumor suppressive effect specific to a patient by determining the presence/absence of the estrogen signal after the addition of said aromatase inhibitor using the method for assessing according to any one of Claims 18 to 20.

22. A method for quantitatively assessing the induction of estrogen signal from a breast cancer tissue, the method comprising the following steps of:
A: slicing the breast cancer tissue in a culture medium and then centrifuging the medium to obtain a supernatant;
B: adding said supernatant to the cell according to any one of Claims 1 to 10;
B: quantifying the reporter protein induced by the supernatant; and
C: quantitatively assessing the induction of estrogen signal from the breast cancer tissue based on the amount of the reporter protein quantified in said step C.

23. A method for quantitatively assessing the induction of estrogen signal from a breast cancer tissue, the method comprising the following steps of:
A: culturing the cell according to any one of Claims 1 to 10 at the bottom of a culture vessel, adding a breast cancer tissue section obtained by slicing the breast cancer tissue to the culture vessel using an insert and co-culturing the cell according to any one of Claims 1 to 10 and said breast cancer tissue section;
B: quantifying the reporter protein induced by said breast cancer tissue section; and
C: quantitatively assessing the induction of estrogen signal from the breast cancer tissue based on the amount of the reporter protein quantified in said step C.

24. A method for cell analysis **characterized in that** the method comprising the steps of;
co-culturing a reporter gene-introduced cell in which a gene coding a reporter protein that specifically responses to a chemical substance (A) is introduced and a chemical substance producing cell that takes in a substrate (B) which is a substrate of the reaction for production of the chemical substance (A), expresses an enzyme (C) that brings conversion to the chemical substance (A), and finally produces and releases the chemical substance (A) into the culture medium, and
analyzing the ability of said chemical substance producing cell to produce said chemical substance (A) based on the expression level of the reporter gene from said reporter gene-introduced cell.

25. The method for cell analysis according to Claim 24 **characterized in that** the method comprises the steps of; culturing said reporter gene-introduced cell at the bottom of a culture vessel, adding a tissue section obtained by slicing a tissue containing the chemical substance producing cell to the culture vessel using an insert and co-culturing said reporter gene-introduced cell and said chemical substance producing cell.

26. A method for cell analysis **characterized in that** the method comprises the steps of;
culturing a reporter gene-introduced cell in which a gene coding a reporter protein that specifically responses to a chemical substance (A) is introduced, with a supernatant obtained by slicing a tissue containing a chemical substance producing cell that takes in a substrate (B) which is a substrate of the reaction for production of the chemical substance (A), expresses an enzyme (C) that brings conversion to the chemical substance (A), and finally produces and releases the chemical substance (A), into a culture medium and centrifuging the medium, and
analyzing the ability of said chemical substance producing cell to produce said chemical substance (A) based on the expression level of the reporter gene from said reporter gene-introduced cell.

27. The method for cell analysis according to any one of Claims 24 to 26 **characterized in that** the ability of said chemical substance producing cell to produce said chemical substance (A) is quantitatively analyzed, using the expression level of the reporter gene obtained by culturing said reporter gene-introduced cell in a culture medium containing a known amount (including zero) of the chamical substance (A) in the absence of said chemical substance producing cell, as the comparative reference.

28. A method for cell analysis **characterized in that** the method comprising the steps of;
co-culturing a reporter gene-introduced cell in which a gene coding a reporter protein that specifically responses to a chemical substance (A) is introduced and a chemical substance producing cell that takes in a substrate (B) which is a substrate of the reaction for production of the chemical substance (A), expresses an enzyme (C) that brings conversion to the chemical substance (A), and finally produces and releases the chemical substance (A) into the culture medium, in the presence of an inhibitor that inhibits said enzyme (C), and
analyzing the effectiveness of said inhibitor based on the expression level of the reporter gene from said reporter gene-introduced cell.

29. The method for cell analysis according to Claim 28 is **characterized in that** the method comprises the steps of; culturing said reporter gene-introduced cell at the bottom of a culture vessel, adding tissue section obtained by slicing a tissue containing said chemical substance producing to the culture vessel using an insert and co-culturing said reporter gene-introduced cell and said chemical substance producing cell in the presence of said inhibitor.

30. A method for cell analysis **characterized in that** the method comprises the steps of;
culturing a reporter gene-introduced cell in which a gene coding a reporter protein that specifically responses to a chemical substance (A) is introduced, with a supernatant obtained by slicing a tissue containing a chemical substance producing cell that takes in a substrate (B) which is a substrate of the reaction for production of the chemical substance (A), expresses an enzyme (C) that brings conversion to the chemical substance (A), and finally produces and releases the chemical substance (A), in a culture medium and centrifuging the medium in the presence of an inhibitor that inhibits said enzyme (C), and
analyzing the effectiveness of said inhibitor based on the expression level of the reporter gene from said reporter gene-introduced cell.

31. The method for cell analysis according to any one of Claims 28 to 30 **characterized in that** the effectiveness of said inhibitor is quantitatively analyzed, using the expression level of the reporter gene obtained by culturing said reporter gene-introduced cell in a culture medium containing a known amount (including zero) of the chamical substance (A) in the absence of said chemical substance producing cell, as the comparative reference.

32. The method for cell analysis according to any one of Claims 24 to 31 **characterized in that** said chemical substance (A) is estrogen, said gene coding a reporter protein is a gene from at least one selected from the group consisting of fluorescent proteins such as GFP and RFP, or from the group consisting of enzymes such as luciferase, β-galactosidase, β-lactamase, and β-glucuronidase, said reporter gene-introduced cell is derived from breast cancer, said substrate (B) is androgen, said enzyme (C) is aromatase, and said chemical substance producing cell is an adipose stromal cell isolated from a breast cancer tissue.

33. The method for cell analysis according to any one of Claims 24 to 31 **characterized in that** said chemical substance (A) is estrogen, said gene coding a reporter protein is a gene from at least one selected from the group consisting of fluorescent proteins such as GFP and RFP, or from the group consisting of enzymes such as luciferase, β-galactosidase, β-lactamase, and β-glucuronidase and the reporter protein coded by said gene has a half-life period of approximately 2 hours to 6 hours, said reporter gene-introduced cell is derived from breast cancer, said substrate (B) is androgen, said enzyme (C) is aromatase, and said substance producing cell is an adipose stromal cell isolated from a breast cancer tissue.

34. The method for cell analysis according to any one of Claims 24 to 31 **characterized in that** said chemical substance (A) is estrogen, said reporter gene-introduced cell is the transgenic cell according to any one of Claims 1 to 10, said substrate (B) is androgen, said enzyme (C) is aromatase, and said chemical substance producing cell is an adipose stromal cell isolated from a breast cancer tissue.

35. The method for cell analysis according to any one of Claims 24 to 34 **characterized in that** the analysis is conducted based on the average value of said expression level of the reporter gene.

36. The method for cell analysis according to any one of Claims 24 to 35 **characterized in that** said expression level of the reporter gene is measured based on an acquired image.

37. The method for cell analysis according to Claim 36 **characterized in that** said reporter gene-introduced cell is cultured to monolayer at the bottom of a culture vessel, said chemical substance producing cell is co-cultured to monolayer on a base having a culture surface at a distance L above said culture vessel bottom, and an image of said reporter gene-introduced cell is acquired from downward of said culture vessel via an objective lens.

38. The method for cell analysis method according to Claim 37 **characterized in that** said base is detachable and said image is acquired after detachment of said base.

39. The method for cell analysis according to Claim 37 **characterized in that** said distance L is not within the range of the focal length of said objective lens and said image is acquired without detachment of said base.

40. The method for cell analysis according to Claim 36 is **characterized in that** the nucleus of said reporter gene-introduced cell is labeled with a spectral property different from that of the introduced reporter protein and said reporter gene-introduced cell is identified by a fluorescent or luminous image of the nuclei through image processing to assess the expression level of the reporter protein.

41. The method for cell analysis according to Claim 40 **characterized in that** the luminance property of a nuclei image is processed to identify the cell in the image, the nucleus region identified through image processing is binary-processed, the fluoresce or luminescence level in the area corresponding to the obtained binary image is measured for each cell to assess the expression level of the reporter gene.

42. The method for cell analysis according to Claim 40 or Claim 41 **characterized in that** the nucleus is labeled with a wavelength longer than the spectral property of the reporter protein.

43. The method for cell analysis according to any one of Claims 32 to 42 **characterized in that** said reporter protein is a green fluorescence protein.

44. The method for cell analysis according to any one of Claims 40 to 43 **characterized in that** the nucleus is labeled with DRAQ5.

45. The method for cell analysis according to any one of Claims 24 to 44 **characterized in that** the amount (including zero) of the chemical substance
(A) or/and said inhibitor to be analyzed is previously quantified and attached inside of the wells of a microplate at the solid state and said chemical substance (A) or/and said inhibitor is melted with a given solvent for the analysis.

46. The method for cell analysis according to any one of Claims 24 to 45 **characterized in that** the expression level of the reporter gene is measured within 24 to 96 hours after the beginning of the co-culture.
